(19) Europäisches Patentamt
European Patent Office
Office européen des brevets

(11) **EP 4 349 295 A1**

(12) **EUROPEAN PATENT APPLICATION**
published in accordance with Art. 153(4) EPC

(43) Date of publication:
**10.04.2024 Bulletin 2024/15**

(21) Application number: **22815334.2**

(22) Date of filing: **02.06.2022**

(51) International Patent Classification (IPC):
**A61B 34/30** (2016.01)     **A61B 34/00** (2016.01)

(52) Cooperative Patent Classification (CPC):
**A61B 34/00; A61B 34/30**

(86) International application number:
**PCT/CN2022/096744**

(87) International publication number:
**WO 2022/253293 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.06.2021 CN 202110614229**
**02.06.2021 CN 202110614231**

(71) Applicant: **Shanghai Microport Medbot (Group) Co., Ltd.**
**Shanghai 201203 (CN)**

(72) Inventors:
• **SONG, Ziwei**
**Shanghai 201203 (CN)**

• **ZHENG, Ayong**
**Shanghai 201203 (CN)**
• **JIANG, Lei**
**Shanghai 201203 (CN)**
• **HE, Chao**
**Shanghai 201203 (CN)**
• **WANG, Jiayin**
**Shanghai 201203 (CN)**
• **ZHANG, Xiaobo**
**Shanghai 201203 (CN)**
• **YAN, Jiaqiang**
**Shanghai 201203 (CN)**

(74) Representative: **Patentship**
**Patentanwaltsgesellschaft mbH**
**Elsenheimerstraße 65**
**80687 München (DE)**

(54) **REMOTE CENTER OF MOTION FOLLOW-UP ADJUSTMENT SYSTEM FOR SUPPORT APPARATUS, INTRAOPERATIVE REMOTE CENTER OF MOTION ADJUSTMENT METHOD, READABLE STORAGE MEDIUM AND SURGICAL ROBOT SYSTEM**

(57)     A remote center of motion follow-up adjustment system for support apparatus includes a locating unit (700) and a control unit (800). The control unit (800) is communicatively connected to the locating unit and configured to acquire, through the locating unit, first positional and postural information of the support apparatus (400) with respect to a patient-side control device (200). The control unit (800) monitors, based on the first positional and postural information, positional and postural variation of the support apparatus (400) with respect to the patient-side control device (200). A robotic arm (210) in the patient-side control device (200) is configured to drive an instrument (220) attached thereto to move while passing through an RCM. The control unit (800) is configured to, when the positional and postural variation is identified to exceed a predetermined value, control the patient-side control device (200) to appropriately move so that the RCM remains unchanged in terms of position and posture with respect to the support apparatus (400). This arrangement allows intraoperative position adjustment without interrupting an ongoing surgical procedure. Also provided are an intraoperative RCM adjustment method, a readable storage medium and a surgical robot system.

Fig. 10

# Description

## TECHNICAL FIELD

The present application relates to the technical field of robot-assisted surgery and, in particular to a remote center of motion follow-up adjustment system for support apparatus, an intraoperative remote center of motion adjustment method, a readable storage medium and a surgical robot system.

## BACKGROUND

The emergence of surgical robots is in line with the development trend toward precision surgery. Surgical robots have become a powerful tool that helps surgeons conduct surgical procedures. To the date, in multiple disciplines and fields, many surgical robots have been developed for various indications.

Designed for accurately and dexterously performing complex surgical operations in a minimally invasive fashion with high precision and high safety, surgical robots have been developed to replace traditional surgery that has been faced with numerous limitations. Differing from the limited human eye, surgical robots can more clearly display internal organs to the operator by using three-dimensional imaging technology. In spaces that would have been inaccessible, robotic arms can perform actions including 360-degree rotation, displacement, swinging and clamping, without shaking. These enable surgical robots to provide patients with the advantages of small incisions, less bleeding and fast recovery, which greatly shorten patients' postoperative hospitalization time and significantly increase their postoperative survival and recovery. Therefore, surgical robots have gained vast popularity among both patients and surgeons. At present, as a kind of high-end medical devices, surgical robots have been widely used in various clinical procedures.

Unlike traditional laparoscopic surgery, a surgical robot system includes remote center of motion (RCM) mechanisms, which can ensure that robotic arms operated by a surgeon always moves about respective RCMs during a surgical procedure. The RCMs coincide with incisions made in a patient's abdomen, ensuring that the robotic arms will not cause damage to the patient during its movement. Despite these benefits, the presence of the RCMs can lead to limitations on an operating space for machinery of the surgical robot. The mechanical volume of a surgical robot is several or even tens of times that of a regular laparoscopic machine. This, coupled with interference among the robotic arms of the surgical robot, further reduces the operating space for the robot's machinery. Consequently, once RCMs of a surgical robot system achieve a match with respective incisions in a patient, any change in the positions of the surgical robot and the patient is no longer allowed, because this may displace one or more of the RCMs and thus lead to possible damage to the patient. All these facts impose very stringent requirements on the positions of incisions made before a surgical robot-assisted procedure begins. For example, if any incision is made in an inappropriate position, an associated robotic arm has to move in a limited space, affecting, or even disabling in severe cases, the progress of the procedure. When this happens, it is necessary to remove instruments and an endoscope from the surgical robot to decouple the RCMs of the surgical robot from the incisions in the patient. After that, the positions of the patient and the surgical robot must be adjusted to again achieve a match between the RCMs of the surgical robot and the incisions in the patient. However, this rematching process is associated with a number of problems, such as interrupting the surgical procedure, costing long time, and disallowing monitoring the adjustment process or checking whether the adjustment positions of the patient and the surgical robot can provide a required operating space. Therefore, this rematching process causes adverse outcomes such as a prolonged surgical time, reduced safety and other factors.

Existing surgical robots require time-consuming preoperative preparation, and the positions of incisions to be made are determined mostly by experience. Moreover, due to differences between individual patients, it is likely that an incision is made in an inappropriate position. Once this occurs, the progress of the procedure may be affected, or the procedure must be interrupted for adjustment of the positions of the surgical robot and the patient. In worse cases, it may be necessary to make one or more new incisions, causing unnecessary damage to the patient. In order to address these operational problems associated with the existing surgical robots, there is an urgent need for a method allowing positional adjustment during a surgical robot-assisted procedure without interrupting it, thereby making the procedure more efficient and safer.

## SUMMARY OF THE INVENTION

It is an object of the present application to provide a remote center of motion follow-up adjustment system for support apparatus, an intraoperative remote center of motion adjustment method, a readable storage medium and a surgical robot system, which overcome the problem of inefficient intraoperative adjustment of positions of a surgical robot and a patient associated with conventional surgical robot systems.

To this end, in a first aspect of the present application, there is provided a remote center of motion follow-up adjustment system for support apparatus, which comprises a locating unit and a control unit,

the control unit communicatively connected to the locating unit, the control unit configured to acquire, through the locating unit, first positional and postural information of the support apparatus with respect to a patient-side control device,

the control unit configured to monitor, based on the first positional and postural information, postural variation of the support apparatus with respect to the patient-side control device, wherein a robotic arm in the patient-side control device is configured to drive an instrument attached thereto to move while passing through an RCM,

the control unit configured to, when the positional and postural variation of the support apparatus with respect to the patient-side control device is identified to exceed a predetermined value, control the patient-side control device to move so that the RCM remains unchanged in terms of position and posture with respect to the support apparatus.

Optionally, the first positional and postural information may comprise a distance of the support apparatus with respect to the patient-side control device and an angle of the support apparatus with respect to the patient-side control device.

Optionally, the system may further comprise a first position and posture acquisition unit communicatively connected to the control unit, the first position and posture acquisition unit configured to acquire second positional and postural information of the robotic arm in the patient-side control device, wherein the control unit derives information of coordinates of the RCM in a coordinate system of the patient-side control device from the second positional and postural information.

Optionally, the system may further comprise a second position and posture acquisition unit communicatively connected to the control unit, the second position and posture acquisition unit configured to acquire third positional and postural information of a supporting board in the support apparatus, wherein the control unit derives information of coordinates of the supporting board in a coordinate system of the support apparatus from the third positional and postural information.

Optionally, the third positional and postural information may comprise information of a lifted/lowered height of the supporting board and a rotation angle of the supporting board, wherein the second position and posture acquisition unit comprises an encoder disposed on a telescopic upright and a gyroscope disposed on a rotary joint, the encoder configured to feed back the information of the lifted/lowered height of the supporting board, the gyroscope configured to feed back the rotation angle of the supporting board.

Optionally, the locating unit may comprise first and second terminals adapted to each other, the first terminal configured to sense a position of the second terminal through a predetermined sensing medium, the first and second terminals disposed on the patient-side control device and/or on the support apparatus depending on a type of the sensing medium and configured to acquire the first positional and postural information of the support apparatus with respect to the patient-side control device.

Optionally, the first terminal may comprise at least two optical photographing devices, wherein the second terminal comprises an optical fiducial marker set comprising at least three non-collinear optical fiducial markers, and wherein the first terminal is disposed on the patient-side control device, and the second terminal is disposed on the support apparatus.

Optionally, the second terminal may comprise a plurality of optical fiducial marker sets each disposed a side edge of the support apparatus.

Optionally, the first terminal may comprise at least two ultrasonic transmitters, wherein the second terminal comprises at least two ultrasonic receivers, and wherein the first terminal is disposed on one of the support apparatus and the patient-side control device, and the second terminal is disposed on the other of the support apparatus and the patient-side control device.

Optionally, each of the ultrasonic receivers may be configured to receive ultrasonic waves transmitted from at least two of the ultrasonic transmitters, wherein the control unit is also configured to discard, based on positional information of the robotic arm in the patient-side control device with respect to the support apparatus, redundant positional information of the ultrasonic transmitters obtained based on the ultrasonic receivers.

Optionally, the first terminal may comprise a magnetic field generator, wherein the second terminal comprises a magnetic locating sensor having at least 3 DoFs, and wherein the first terminal is disposed on the patient-side control device, and the second terminal is disposed on the support apparatus.

Optionally, the second terminal may comprise a magnetic locating sensor having at least 6 DoFs.

Optionally, the first terminal may comprise a magnetic field generator, wherein the second terminal comprises at least three non-collinear magnetic locating sensors, and wherein the first terminal is disposed on the patient-side control device, and the second terminal is disposed on the support apparatus.

Optionally, the first terminal may comprise a laser, wherein the second terminal comprises a photographing device, and wherein both the first and second terminals are disposed on the patient-side control device, or both the first and second terminals are disposed on the support apparatus.

Optionally, the locating unit may further comprise a reflector plate disposed on the one of the patient-side control device and the support apparatus, on which the laser and the photographing device are not disposed, and configured to reflect laser radiation emitted from the laser.

To the above end, in a second aspect of the present application, there is provided a surgical robot system comprising a support apparatus, a patient-side control device and the remote center of motion follow-up adjustment system for support apparatus as defined above. The remote center of motion follow-up adjustment system for support apparatus is configured to control the patient-side control device to move so that an RCM remains unchanged in terms of position and posture with respect to the support apparatus.

To the above end, in a third aspect of the present application, there is provided an intraoperative remote center of motion adjustment method, which comprises:

during movement of an instrument attached to a robotic arm in a patient-side control device relative to a guide tube disposed around the instrument, determining a force acting on the instrument and/or a force acting on the guide tube, wherein the instrument attached to the robotic arm is configured to move while passing through an RCM; and according to the determined force on the instrument and/or force on the guide tube, adjusting a position and posture of the robotic arm so that the RCM remains unchanged in terms of position and posture with respect to a support apparatus.

Optionally, determining the force acting on the instrument may comprise:

determining torques on joints in the patient-side control device; and deriving the force acting on the instrument from the torques on the joints.

Optionally, determining the force acting on the instrument may comprise:

acquiring a force detected by a torque sensor disposed on a base of the patient-side control device to derive an external force acting on the robotic arm; and deriving the force acting on the instrument from the external force acting on the robotic arm.

Optionally, determining the force acting on the guide tube may comprise:

acquiring a force signal detected by a force sensor disposed on the guide tube; and obtaining the force acting on the guide tube based on the force signal.

Optionally, adjusting the position nd posture of the robotic arm according to the determined force on the guide tube may comprise:
according to the force on the guide tube, adjusting the position and posture of the robotic arm numerically, analytically, or using robot kinematics.

Optionally, adjusting the posture of the robotic arm numerically may comprise:

acquiring position and postural information of the RCM and positional information of joints in the robotic arm; based on the postural information of the RCM and the positional information of the joints, calculating a plurality of adjustment paths for the robotic arm according to a predefined algorithm; and screening for a desired one of the adjustment paths for the robotic arm.

Optionally, the predefined algorithm may comprise:
for each joint, from a magnitude of the force acting on the guide tube, a range of movement across an entire travel of the joint, a step count of a motor associated with the joint and a threshold for step size amplification, an iterative step size is calculated for the joint; and thereby deriving all possible adjustment paths for the robotic arm.

Optionally, screening for the desired adjustment path for the robotic arm may comprise:

subjecting one or more of the possible adjustment paths for the robotic arm to calculation using a convergence determining function.
Optionally, the convergence determining function may be subject to conditions including convergence conditions and constraint conditions,
the convergence conditions including coincidence of a moving direction of an end of the robotic arm with a direction of the force acting on the guide tube and no increase in a force acting on the end of the robotic arm,
the constraint conditions including at least one of:
no collision between robotic arms, a position and postural change of the end of the robotic arm less than a predetermined threshold, and positions of the joints lying within a predetermined position range.

Optionally, screening for the desired adjustment path for the robotic arm further may comprise:
subjecting all the possible adjustment paths for the robotic arm to calculation using a cost function, a heuristic function and weighting function.

Optionally, the method may further comprise, after the position and posture of the robotic arm is adjusted according to the determined force on the instrument and/or force on the guide tube,

according to the adjusted position and posture of the robotic arm, adjusting the instrument attached to the robotic arm to a desired position and posture; and
synchronizing a position and posture of a control manipulator in a surgeon's control terminal to the position and posture of the robotic arm.

To the above end, in a fourth aspect of the present application, there is provided a readable storage medium storing thereon a program, which, when executed, implements the intraoperative remote center of motion adjustment method as defined above.

To the above end, in a fifth aspect of the present application, there is provided a RCM adjustment system comprising a sensing unit, an actuation unit and a control unit, the actuation unit comprising a robotic arm configured for attachment thereto of an instrument inserted through a guide tube, the sensing unit configured to sense a force acting on the instrument and/or a force acting on the guide tube, the control unit communicatively connected to both the sensing unit and the actuation unit and configured to control positional and postural adjustment of the robotic arm according to the intraoperative remote center of motion adjustment method as defined above so that an RCM remains unchanged in terms of position and posture with respect to a support apparatus.

Optionally, the system may further comprise a detection unit, which is communicatively connected to the control unit and configured to detect positional and postural variation information of the actuation unit, wherein the control unit is configured to provide closed-loop control over the actuation unit and the detection unit.

To the above end, in a sixth aspect of the present application, there is provided a surgical robot system comprising a support apparatus, a guide tube and the remote center of motion adjustment system as defined above, wherein the instrument attached to the robotic arm in the remote center of motion adjustment system is configured to be inserted through the guide tube, and wherein the control unit in the remote center of motion adjustment system is configured to adjust a position and posture of the robotic arm so that an RCM remains unchanged in terms of position and posture with respect to the support apparatus.

In summary, the present application provides a remote center of motion follow-up adjustment system for support apparatus, which includes a locating unit and a control unit. The control unit is communicatively connected to the locating unit and configured to acquire, through the locating unit, first positional and postural information of the support apparatus with respect to a patient-side control device. The control unit monitors, based on the first positional and postural information, positional and postural variation of the support apparatus with respect to the patient-side control device. A robotic arm in the patient-side control device is configured to drive an instrument attached thereto to move while passing through an RCM. The control unit is configured to, when the positional and postural variation of the support apparatus with respect to the patient-side control device is identified to exceed a predetermined value, control the patient-side control device to move so that the RCM remains unchanged in terms of position and posture with respect to the support apparatus. The application also provides an intraoperative RCM adjustment method, which comprises: during movement of an instrument attached to a robotic arm in a patient-side control device relative to a guide tube disposed around the instrument, determining a force acting on the instrument and/or a force acting on the guide tube, wherein the instrument attached to the robotic arm is configured to move while passing through an RCM; and according to the determined force on the instrument and/or force on the guide tube, adjusting a position and posture of the robotic arm so that the RCM remains unchanged in terms of position and posture with respect to a support apparatus.

With this configuration, through the locating unit, the control unit can monitor in real time relative positions and postures of the support apparatus and the patient-side control device and can control the patient-side control device to move appropriately so that the RCM remains positionally and posturally unchanged with respect to the support apparatus. In addition, intraoperative RCM adjustment can be made so that the position and posture of the robotic arm is adjusted according to the determined force on the instrument and/or force on the guide tube. This enables real-time adjustment of the robotic arm according to the adjustment of the RCM, ensuring that the RCM remains positionally and posturally unchanged with respect to the support apparatus. The intraoperative position adjustment can be made without interrupting the ongoing surgical procedure, addressing a limited space for movement of robotic arms, non-ideal incision positions and other problems associated with the prior art, which arises from a robot/patient positional relationship. Moreover, various forms of intraoperative position adjustment can be made in an efficient fashion, without requiring instrument detachment, thereby improving the surgical robot's efficiency and safety, shortening the time required for preoperative preparation, effectively reducing the risk and drawbacks associated with the conventional incision-making practice, increasing surgical accuracy, reducing patient trauma and pain and facilitating patient recovery.

**BRIEF DESCRIPTION OF THE DRAWINGS**

Those of ordinary skill in the art would appreciate that the accompanying drawings are provided to facilitate a better understanding of the present invention and do not limit the scope thereof in any sense, in which:

Fig. 1 schematically depicts a surgical scene associated with a surgical robot system according to the present application;
Fig. 2 is an overall flowchart of steps in a surgical plan according to the present application;
Fig. 3 schematically depicts establishment of a world coordinate system for a surgical scene according to the present application;
Figs. 4a and 4b schematically depicts surgical scene establishment according to the present application;
Fig. 5 schematically depicts determination of incisions according to the present application;
Fig. 6 is a schematic depiction of a patient-side surgical platform according to the present application;
Fig. 7a schematically depicts how a safety zone is defined using a position sensor according to the present application;
Fig. 7b schematically depicts how a safety zone is defined using a fiber-optic shape sensor according to the present application;
Fig. 8 schematically depicts a robot before it makes self-adaptive adjustment according to the present application;
Fig. 9 schematically depicts a robot after it makes self-adaptive adjustment according to the present application;
Fig. 10 is a schematic depiction of a surgical robot system according to an embodiment of the present application;
Fig. 11 schematically depicts a photographing and locating assembly included in a locating unit according to an embodiment of the present application;
Figs. 12a and 12b schematically depict how location is achieved using the binocular vision principles according to an embodiment of the present application;
Fig. 13 schematically depicts scattered optical fiducial marker sets according to an embodiment of the present application;
Fig. 14 schematically depicts integrated optical fiducial marker sets according to an embodiment of the present application;
Figs. 15a and 15b schematically depict an ultrasonic locating assembly included in a locating unit according to an embodiment of the present application;
Fig. 16 schematically depicts how location is achieved by an ultrasonic locating assembly according to an embodiment of the present application;
Fig. 17 schematically depicts a magnetic locating assembly included in a locating unit according to an embodiment of the present application;
Figs. 18a and 18b schematically depict another magnetic locating assembly included in a locating unit according to an embodiment of the present application;
Figs. 19a and 19b schematically depict a laser locating assembly included in a locating unit according to an embodiment of the present application;
Fig. 20 schematically depicts a laser locating assembly according to an embodiment of the present application;
Fig. 21 schematically depicts a measurement method based on laser reflection according to an embodiment of the present application;
Fig. 22 schematically depicts a first position and posture acquisition unit according to an embodiment of the present application;
Fig. 23 schematically depicts a second position and posture acquisition unit according to an embodiment of the present application;
Fig. 24 schematically depicts a patient-side control device and a support apparatus according to an embodiment of the present application, which are placed in a same horizontal plane;
Fig. 25 is a flowchart of an intraoperative RCM adjustment method according to an embodiment of the present application;
Fig. 26 schematically depicts an instrument inserted through a trocar according to an embodiment of the present application;
Figs. 27a and 27b schematically depict an intraoperative RCM adjustment system according to an embodiment of the present application;
Fig. 28 schematically depicts a mechanism RCM according to an embodiment of the present application;
Fig. 29 schematically depicts deriving a force acting on an instrument from torques on joints according to an embodiment of the present application;

Fig. 30 schematically depicts determining a force acting on an instrument using a torque sensor according to an embodiment of the present application;

Fig. 31 schematically depicts a trocar fixation assembly according to an embodiment of the present application;

Fig. 32 is a diagram showing mechanic analysis of a trocar according to an embodiment of the present application;

Fig. 33 schematically depicts determining a force acting on a trocar with a 3D force sensor according to an embodiment of the present application;

Figs. 34a and 34b schematically depict an operating space for an instrument before and after it is adjusted according to an embodiment of the present application;

Figs. 35a and 35b schematically depict a patient before and after his/her position is adjusted according to an embodiment of the present application;

Fig. 36 schematically depicts an adjustment confirmation step according to an embodiment of the present application;

Fig. 37 schematically depicts an indication displayed for confirmation of adjustment according to an embodiment of the present application;

Figs. 38a and 38b schematically depict indications provided in an adjustment process according to an embodiment of the present application;

Fig. 39 schematically depicts an instrument retracted back into a trocar according to an embodiment of the present application;

Fig. 40 schematically depicts an iterative algorithm according to an embodiment of the present application;

Fig. 41 schematically depicts rules of movements for joints according to an embodiment of the present application; and

Fig. 42 schematically depicts a displayed indication indicative of the completion of adjustment according to an embodiment of the present application.

In these figures,

100 denotes a surgeon-side control device; 101, a master manipulator; 102, an imaging device; 103, a foot pedal surgical control device;

200, a patient-side control device; 201, a base; 210, a robotic arm; 211, an adjustment arm; 212, an instrument arm; 220, an instrument; 221, a surgical instrument; 222, an endoscope;

300, an image cart; 302, a display device; 400, a support apparatus; 410, a patient; 411, an incision; 500, a safety zone; 510, an operating space; 520, a lesion area; 610, a position sensor; 620, a fiducial marker; 630, a fiber-optic shape sensors;

700, a locating unit; 711, an optical photographing device; 712, an ultrasonic transmitter; 7120, a redundant solution; 713, a magnetic field generator; 714, a laser; 721, an optical fiducial marker set; 7210, an optical fiducial marker; 722, an ultrasonic receiver; 723, a magnetic locating sensor; 724, a photographing device; 730, a reflector plate;

800, a control unit; 810, a first position and posture acquisition unit; 820, a second position and posture acquisition unit; 821, a telescopic upright; 822, a rotary joint;

900, a trocar; 910, a detection unit; 920, an actuation unit; 930, a control unit; 940, a trocar fixation assembly; 941, a fixation member; 942, a passive joint; 943, a force sensor; 944, an attachment member; and 950, a sensing unit.

## DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, the structures shown in the figures are usually part of actual structures. In particular, as the figures tend to have distinct emphases, they are often drawn to different scales.

As used herein, the singular forms "a", "an" and "the" include plural referents. Herein, the term "or" is generally employed in the sense of "and/or", "several" of "at least one", and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a lesion in a patient. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

It is an object of the present application to provide a remote center of motion (RCM) follow-up adjustment system for support apparatus, an intraoperative RCM adjustment method, a readable storage medium and a surgical robot system, which overcome the problem of impossibility of intraoperative adjustment of positions of a surgical robot and a patient associated with conventional surgical robot systems.

The invention will be described below with reference to the accompanying drawings.

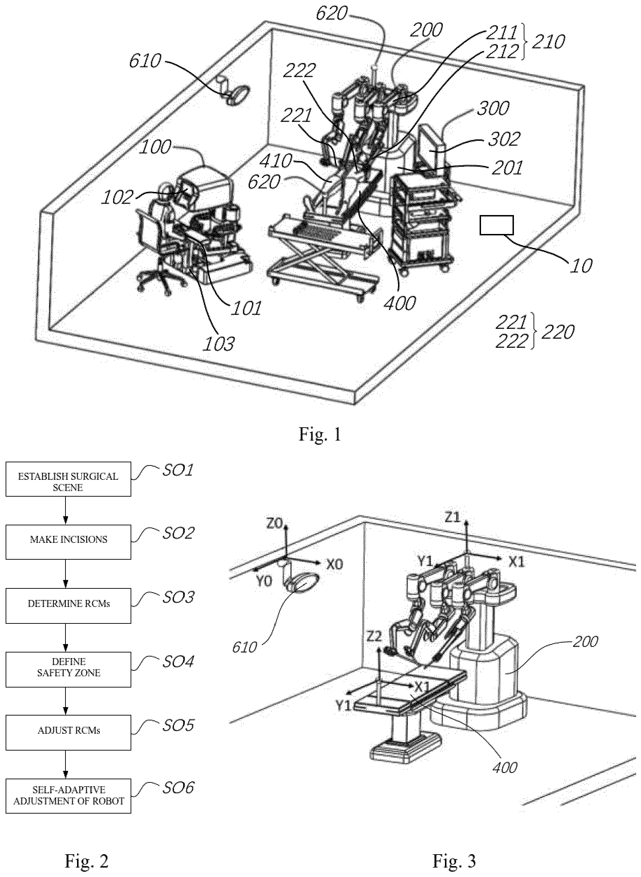

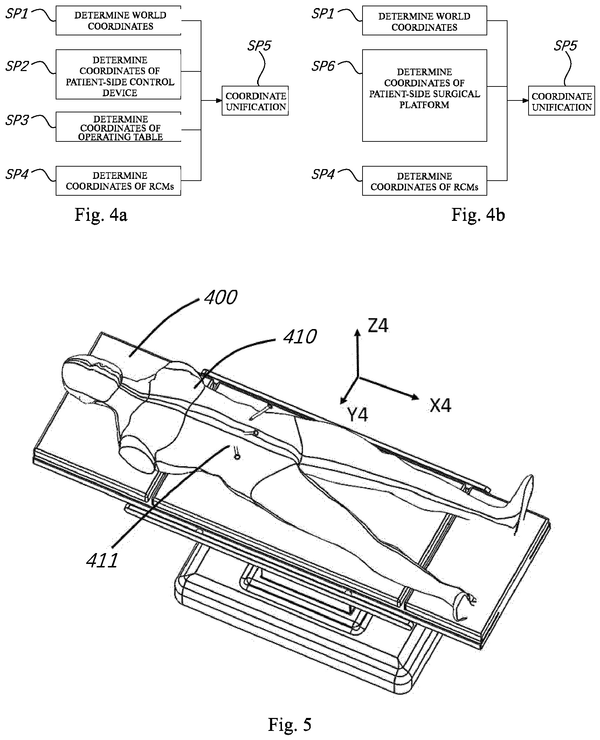

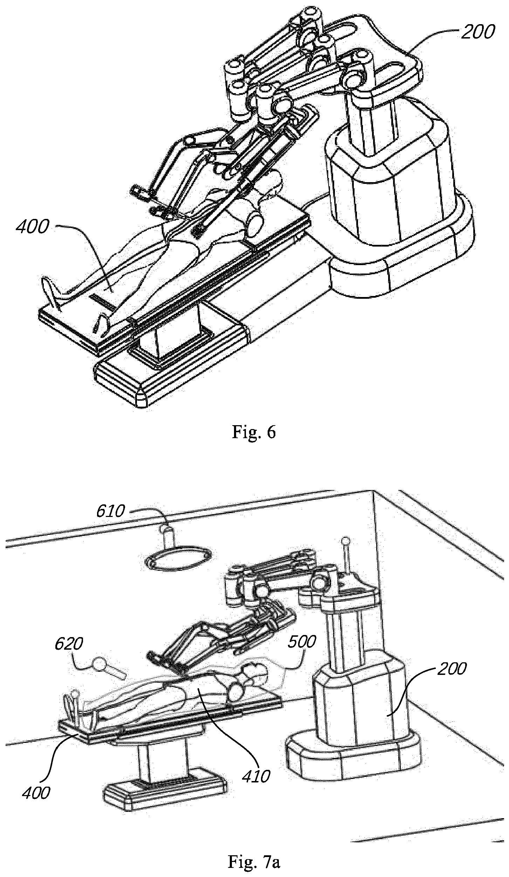

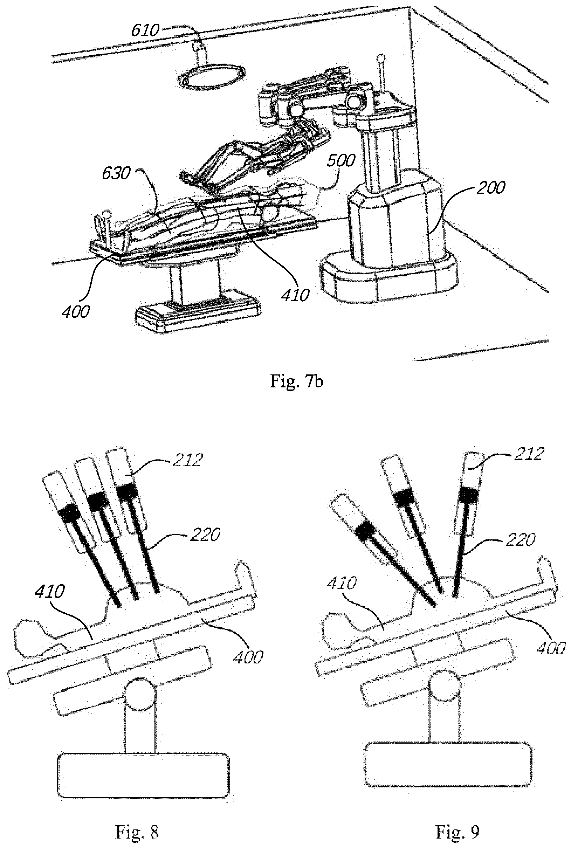

Reference is made to Figs. 1 to 9 below. Fig. 1 schematically illustrates a surgical scene in which a surgical robot system according to the present application can be used. Fig. 2 is an overall flowchart of steps in a surgical plan according to the present application. Fig. 3 schematically depicts establishment of a world coordinate system for a surgical scene according to the present application. Figs. 4a and 4b schematically depicts surgical scene establishment according to the present application. Fig. 5 schematically depicts determination of incisions according to the present application. Fig. 6 is a schematic depiction of a patient-side surgical platform according to the present application. Fig. 7a schematically depicts how a safety zone is defined using a position sensor according to the present application. Fig. 7b schematically depicts how a safety zone is defined using a fiber-optic shape sensor according to the present application. Fig. 8 schematically depicts a robot before it makes self-adaptive adjustment according to the present application. Fig. 9 schematically depicts a robot after it makes self-adaptive adjustment according to the present application. Fig. 1 shows an application scenario of a surgical robot system including a surgical robot teleoperated in a master/slave style. The surgical robot system includes a surgeon-side control device 100, a patient-side control device 200, a master controller 10 and a support apparatus 400 (e.g., an operating table) for supporting a subject on which a surgical procedure is to be performed. It is to be noted that, in some embodiments, the support apparatus 400 may be replaced with other surgical operating platforms, without departing from the scope of the present application.

The surgeon-side control device 100 is a terminal device which can be manipulated to teleoperate the surgical robot. It includes master manipulators 101 mounted thereon, which can receive information about an operator's hand motions. This information is taken as input motion control signals for the whole system. Optionally, the master controller may also be provided on the surgeon-side control device 100. Preferably, the surgeon-side control device 100 further includes an imaging device 102 capable of providing the operator with three-dimensional (3D) images, which can provide information necessary for the surgical procedure to be performed. This information may include the type, number and intra-abdominal position and posture of surgical instruments, the shape and arrangement of the diseased and surrounding organs or tissue, and the like. Optionally, the surgeon-side control device 100 may further includes a foot pedal surgical control device 103 for the operator to input commands for electroresection, electrocoagulation and other surgical maneuvers.

The patient-side control device 200 is an implementation platform for teleoperation of the surgical robot, which includes a base 201 and a surgical instrument assembly mounted thereon. The surgical instrument assembly includes robotic arms 210 and instruments 220. The instruments 220 include a surgical instrument 221 for performing a specific maneuver (e.g., a high-frequency electrotome, etc.) and an endoscope 222 for assistance in observation. In one embodiment, each robotic arm includes an adjustment arm 211 and an instrument arm 212. The instrument arm 212 is a mechanical RCM mechanism for driving an instrument 220 to move about a mechanical RCM to effect minimally invasive surgical treatment of a patient 410 lying on the support apparatus 400. The adjustment arm 211 is configured to adjust the position of the mechanical RCM within an operating space. In another embodiment, each robotic arm 210 is a mechanism with at least six degrees of freedom (DoFs) configured to drive an instrument 220 to move about an active RCM under the control of a program. The instruments 220 are used to perform specific surgical maneuvers such as clamping, dissecting and cutting, or serve as a surgical aid for taking photo, for example. It is to be noted that since the instrument 220 actually has a certain volume, the aforementioned "RCM" should be understood as a region. Of course, those skilled in the art can understand the meaning of "RCM" based on the common general knowledge in the art.

The master controller is communicatively connected to both the surgeon-side control device 100 and the patient-side control device 200 and is configured to control motions of the surgical instrument assembly according to motions of the master manipulators 101. Specifically, the master controller includes a master/slave mapping module configured to obtain positions and postures of ends of the master manipulators 101 and a predetermined master/slave mapping relationship so as to obtain desired positions and postures of ends of the surgical instrument assembly. In this way, the master controller 10 is able to control the robotic arms 210 to drive the instruments 220 to move to the desired positions and postures. The master/slave mapping module is also configured to receive commands for functions and maneuvers intended to be performed by the instruments (e.g., electroresection, electrocoagulation and other necessary maneuvers) and accordingly control associated energy drivers of the instruments 220 to release energy required by the electroresection, electrocoagulation and other surgical maneuvers.

The medical robot system also includes an image cart 300 including an endoscopic processor (not shown) communicatively connected to the endoscope 222. The endoscope 222 is configured to collect information about the surgical procedure to be performed within a body cavity (of the patient), and the endoscopic processor is configured to visualize the surgical procedure information obtained by the endoscope 222 and transmit it to the imaging device 102, allowing the operator to view the surgical procedure information. Optionally, the image cart 300 may further include a display device 302, which is communicatively connected to the endoscopic processor and configured to display the surgical procedure information in real time to an assistant operator (e.g., a nurse).

During the surgical procedure, while sitting in front of the surgeon-side control device 100, which is located outside a sterile area, the operator (e.g., a main operating surgeon) may view the surgical procedure information displayed on the imaging device 102 and control movement of the surgical instrument assembly and a laparoscope by manipulating the master manipulators 101, thereby performing various surgical maneuvers.

Referring now to Fig. 2, an application scenario of the surgical robot system provided herein will be exemplified below. Before RCM adjustment, or adjustment of the patient-side control device, is made in the surgical robot system, the steps as follows may be carried out.

Step SO1: Establish a surgical scene and transform all the robotic arms 210 from a coordinate system of the support apparatus into a world coordinate system. In this way, positions and postures of the support apparatus 400 and the robotic arms 210 can be all described by the world coordinate system. Referring to Fig. 3, in one example, some means may be used to establish the world coordinate system $(X0,Y0,Z0)$ for the surgical scene and to transform coordinates $(X1,Y1,Z1)$ of the surgical robot and coordinates $(X2,Y2,Z2)$ of the support apparatus into the world coordinate system $(X0,Y0,Z0)$ so that they can all described by coordinates in the coordinate system of the surgical scene. A positional relationship between the coordinates of the support apparatus and the coordinates of the surgical robot is established as a basis for subsequently determining changes in the positions of incisions to be made in the patient and in RCM positions of the patient-side control device 200 caused by any adjustment made by the support apparatus 400. The establishment of the surgical scene is an initial step in intraoperative RCM adjustment. In one example, the relative positional relationship between the patient-side control device 200 and the support apparatus 400 may be established using a position sensor 610 (e.g., a binocular vision apparatus) and a fiducial marker 620. As shown in Fig. 4a, the establishment of the surgical scene may primarily include the steps as follows:

Step SP1: Determine world coordinates. The position sensor 610 may be used to determine coordinates $(X0,Y0, Z0)$ of the patient-side control device 200 and the support apparatus 400 in a world where they are present, enabling coordinate unification of the systems.

Step SP2: Determine coordinates of the patient-side control device. According to the arrangement of the robotic arms 210 in the surgical robot of the patient-side control device 200, the position sensor 610 may be used to determine coordinates of the patient-side control device 200 in the world coordinate system $(X0,Y0,Z0)$, which identify the position of the patient-side control device 200 in the world coordinate system $(X0,Y0,Z0)$ and can be subsequently used to determine the positions of the RCMs of the surgical robot system in the world coordinate system.

Step SP3: Determine coordinates of the support apparatus. The position sensor 610 may be used to determine coordinates of the support apparatus 400 in the world coordinate system, which identify the position of the support apparatus 400 in the world coordinate system and can be subsequently used to determine coordinates of the incisions to be made in the patient after they are moved due to any adjustment made to the support apparatus 400, as well as corresponding travel paths thereof.

Step SP4: Determine coordinates of the RCMs. As shown in Fig. 5, after the patient 410 has been placed on the support apparatus 400 and the positions of the incisions 411 have been marked, the position sensor 610 may be used to determine coordinates of the incision marks 411 on the skin of the patient 410 in the world coordinate system, which identify the positions of the incisions 411 to be made in the world coordinate system.

Step SP5: Achieve coordinate unification. Coordinate unification can be achieved after the determination of world coordinates in step SP1, the determination of coordinates of the patient-side control device in step SP2, the determination of coordinates of the support apparatus in step SP3 and the determination of coordinates of the RCMs in step SP4 are completed. In this way, intraoperative adjustment can be made in the same single coordinate system.

Depending on the configuration of the surgical robot, the determination of world coordinates can be carried out in different manners. For example, in another embodiment, the support apparatus 400 is integrated with the patient-side control device 200 to form an integral patient-side surgical platform, as shown in Fig. 6. As would be appreciated, in this case, it may be unnecessary to separately determine coordinates of the patient-side control device 200 and the support apparatus 400, and steps SP2 and SP3 may be replaced with step SP6 for determining coordinates of the patient-side surgical platform. A flowchart of a process for surgical scene establishment in this case is as shown in Fig. 4b. Of course, the present application is not limited to the above-described coordinate determination method, and those skilled in the art may select any other suitable coordinate determination method as practically desired.

Step SO2: Make the incisions. With continued reference to Fig. 2, the operator may determine the positions of the incisions according to the position of a target lesion in the position, and the incisions may be then made at the determined positions.

Step SOS: Determine RCMs. After the incisions are made, some technical means may be used to identify the incisions made in the body of the patient and determine their coordinates in the world coordinate system. For example, the coordinates of the incisions may be determined using the position sensor 610 and the fiducial marker 620. The coordinates of the incisions may vary when adjustment is made to the support apparatus 400. The coordinates of the RCMs of the patient-side control device 200 are determined so as to match the coordinates of the incisions, and the matching may be monitored intraoperatively to ensure safety of the surgical procedure.

Further, according to this embodiment, the RCM determination may be accomplished according to either of the two methods described below.

In a first method, the position sensor 610 is used to determine the RCMs in the world coordinate system. After the incisions 411 are made, the fiducial marker 620 (serving as a locating reference) is used to determine the coordinates of the incisions 411 (i.e., the coordinates of the RCMs). Specifically, the fiducial marker 620 is (physically) attached to the support apparatus 400 so that, as long as the patient 410 remains stationary relative to the support apparatus 400, any change in the coordinates of the RCMs can only be caused by movement of the support apparatus 400.

In a second method, the position sensor 610 is used to determine the RCMs in the world coordinate system on a real-time basis. The fiducial marker 620 serving as a reference for coordinate determination is fixed at a position relative to the incisions 411 in the patient 410 in a certain way (e.g., by adhesive bonding), and coordinates of the fiducial marker 620 in the world coordinate system are determined in real time. In this case, the coordinates of the fiducial marker 620 will vary with real-time changes of the support apparatus 400 and the patient 410. Thus, the coordinates for the position of the incisions 411 in the patient 410 can be more accurately determined.

Step SO4: Define a safety zone 500. This step is an optional step included in some embodiments. After the patient is immobilized on the support apparatus 400, his/her coordinates are determined, in order to prevent collision of any robotic arm 210 with the patient during the surgical procedure or intraoperative adjustment and thereby ensure safety of the patient. In particular, defining the safety zone 500 may include the steps as follows. Step SO41: Obtain body surface information of a subject of interest (e.g., the patient 410) on the support apparatus 400. Step SO42: Define the safety zone 500 based on the body surface information and correlate positional information of the safety zone 500 with positional information of the support apparatus 400 so that the robotic arms 210 will not enter the safety zone 500. In practice, the safety zone 500 may stretch from the body surface of the patient out a predetermined distance, and the robotic arms 210 should avoid the safety zone 500 during its movement in order to avoid causing damage to the patient.

Referring to Fig. 7a, in an alternative embodiment, the safety zone may be defined using the position sensor 610 and the fiducial marker 620 according to a method including: bringing the fiducial marker 620 into contact with the body surface of the subject of interest at various locations and capturing point cloud data with the position sensor 610; and defining the safety zone by fitting the point cloud data. Referring to Fig. 7b, in another alternative embodiment, the safety zone may also be defined using a fiber-optic shape sensor 630 according to a method including: capturing shape data with the fiber-optic shape sensor 630 attached to the body surface of the subject of interest; and defining the safety zone by fitting the shape data.

Step SOS: Adjust the RCMs during the procedure to enable an operating space of the surgical robot to accommodate an intended maneuver.

Step SO6: Make self-adaptive adjustment by the robot. This step is an optional step included in some embodiments. After the RCM adjustment in step SO5 is completed, the robotic arms 210 may additionally adjust themselves to positions and postures favorable to operation, in particular based on the position of the target lesion, positions and postures of the RCMs and a relative positional relationship between the safety zone and the robotic arms 210. In practice, after the RCM adjustment in step SO5 is completed, the positions and postures of the robotic arms 210 may be not necessarily suitable for operation. In this case, as shown in Figs. 8 and 9, the robot may make self-adaptive adjustment, which tunes the robotic arms 210 to positions and orientations suitable for operation of the instruments 220. After the robotic arms 210 have been adjusted to the desired positions and postures, positions and postures of the control arms (i.e., the master manipulators 101) of the surgeon's console (i.e., the surgeon-side control device 100) may be synchronized and updated to the positions and postures of the respective robotic arms 210.

As noted in the Background section, for a conventional surgical robot system, once a match has been obtained between its RCMs and incisions in a patient, any further adjustment to the position of the surgical robot or to that of the patient is not allowed. Otherwise, the positions of the RCM may shift, possibly leading to damage to the patient. In view of this, a few embodiments are provided herein to address the challenge of intraoperative adjustment to a patient's position.

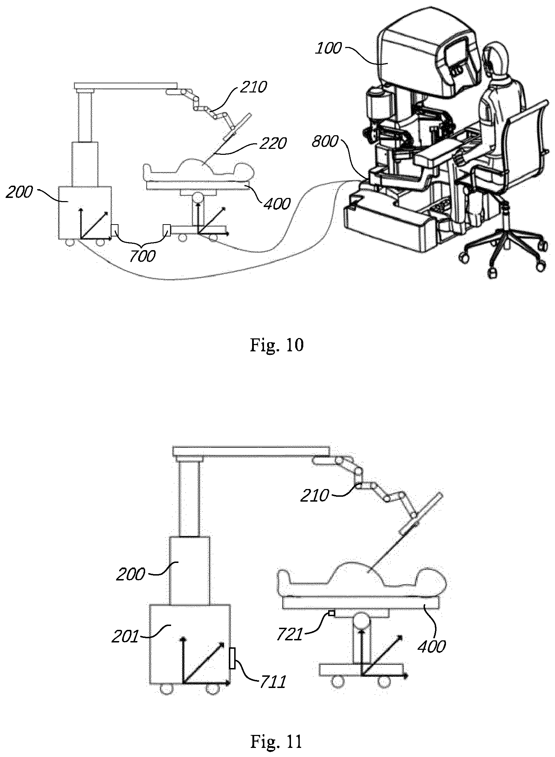

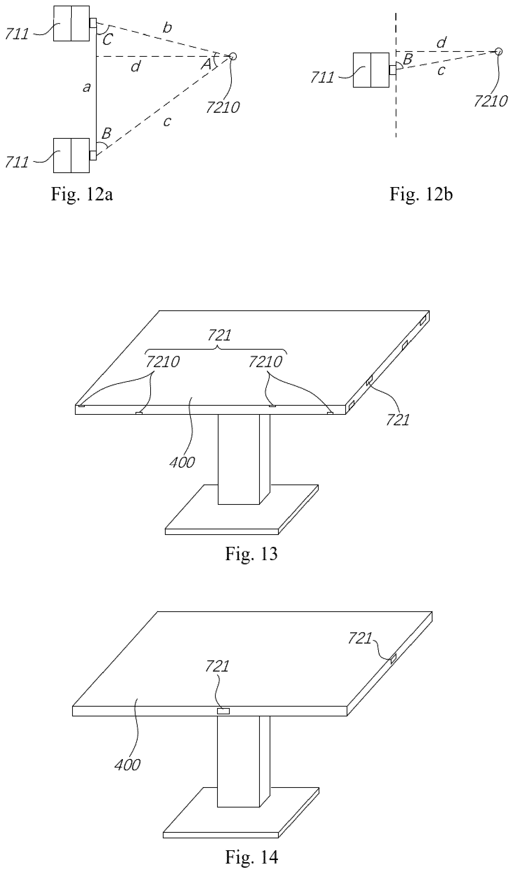

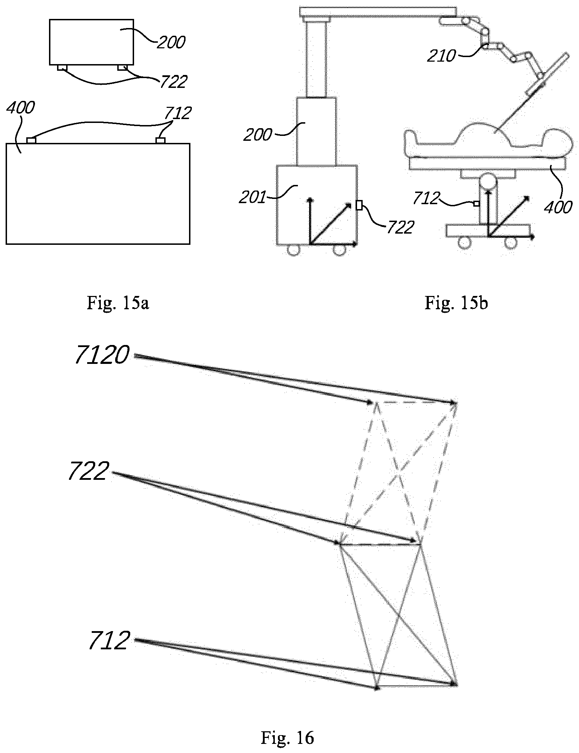

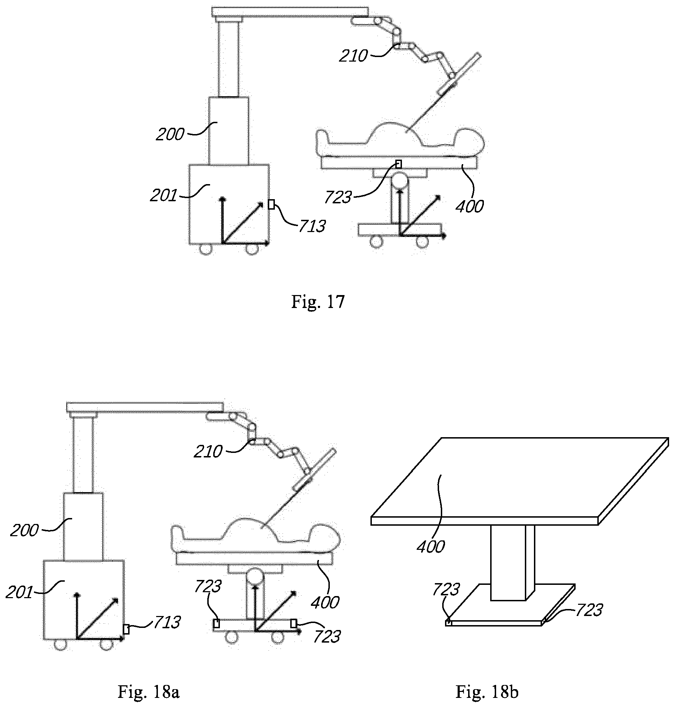

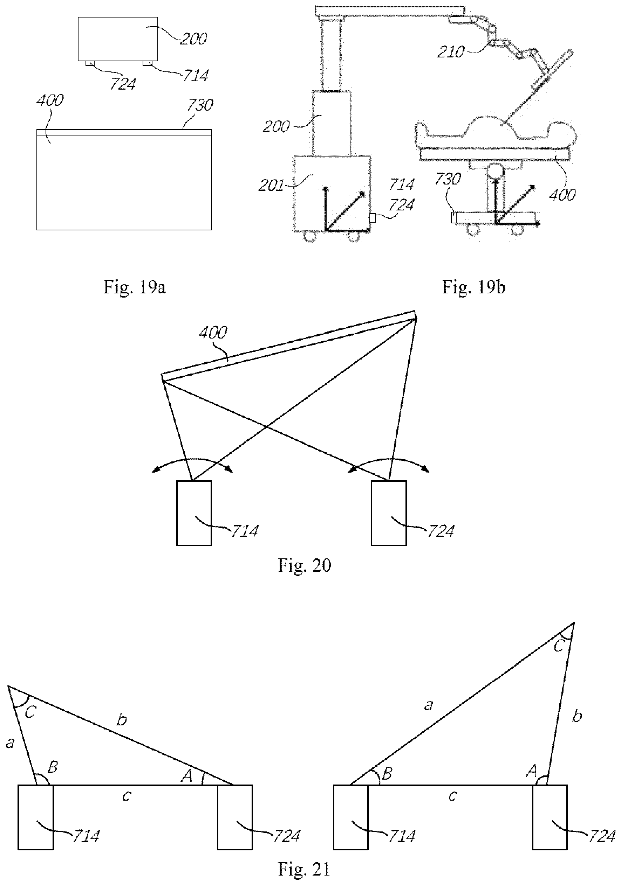

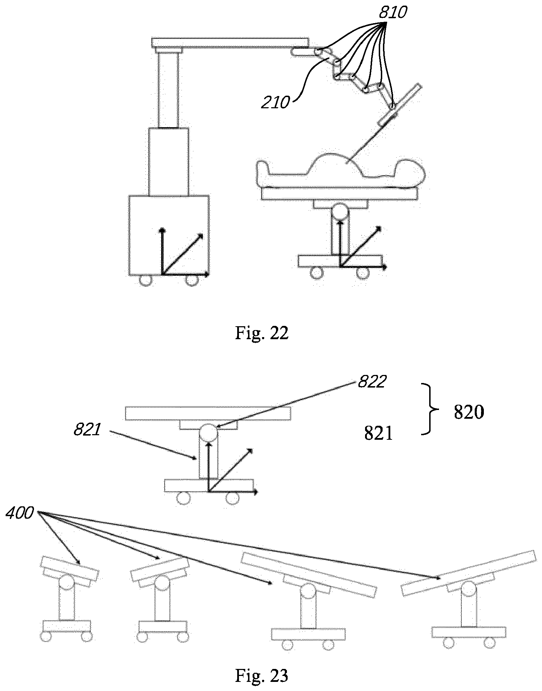

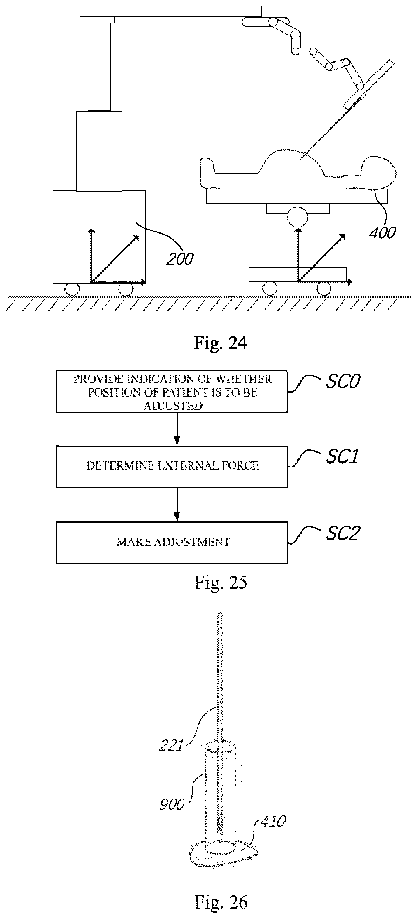

Reference is made to Figs. 10 to 24 below. Fig. 10 is a schematic depiction of a surgical robot system according to an embodiment of the present application. Fig. 11 schematically depicts a photographing and locating assembly in a locating unit according to an embodiment of the present application. Figs. 12a and 12b schematically depicts how location is achieved using the binocular vision principles according to an embodiment of the present application. Fig. 13 schematically depicts scattered optical fiducial marker sets according to an embodiment of the present application. Fig. 14 schematically depicts integrated optical fiducial marker sets according to an embodiment of the present application. Figs. 15a and 15b schematically depict an ultrasonic locating assembly in a locating unit according to an embodiment of the present application. Fig. 16 schematically depicts how location is achieved by an ultrasonic locating assembly according to an embodiment of the present application. Fig. 17 schematically depicts a magnetic locating assembly included in a locating unit according to an embodiment of the present application. Figs. 18a and 18b schematically depict another magnetic locating assembly included in a locating unit according to an embodiment of the present application. Figs. 19a and 19b schematically depict a laser locating assembly included in a locating unit according to an embodiment of the present application. Fig. 20 schematically depicts a laser locating assembly according to an embodiment of the present application. Fig. 21 schematically depicts a measurement method based on laser reflection according to an embodiment of the present application. Fig. 22 schematically depicts a first position and posture acquisition unit according to an embodiment of the present application. Fig. 23 schematically depicts a second position and posture acquisition unit according to an embodiment of the present application. Fig. 24 schematically depicts a patient-side control device and a support apparatus according to an embodiment of the present application, which are placed in the same horizontal plane.

Referring to Fig. 10, in one embodiment of the present application, there is provided a remote center of motion follow-up adjustment system for support apparatus. The system includes a locating unit 700 and a control unit 800 communicatively connected to the locating unit 700. The control unit 800 is configured to use the locating unit 700 to obtain first positional and postural information of the support apparatus 400 with respect to a patient-side control device 200 and, based on the first positional and postural information, monitor variation in the position and posture of the support apparatus 400 with respect to the patient-side control device 200. A robotic arm 210 in the patient-side control device 200 is configured to drive an instrument 220 attached thereto to move while passing through an RCM. The control unit 800 is configured to, when identifying an amount of variation in the position and posture of the support apparatus 400 with respect to the patient-side control device 200, which exceeds a predetermined value, control the patient-side control device 200 to move responsively so that the RCM remains positionally and posturally unchanged relative to the support apparatus 400.

With this arrangement, the control unit 800 can utilize the locating unit 700 to monitor in real time the position and posture of the support apparatus 400 with respect to the patient-side control device 200 and can control the patient-side control device 200 to move so as to keep the RCM positionally and posturally unchanged relative to the support apparatus 400. This enables intraoperative position adjustment to be done without interrupting the ongoing surgical produce, addressing a limited space for movement of robotic arms, non-ideal incision positions and other problems associated with the prior art, which arises from a robot/patient positional relationship. Moreover, various forms of intraoperative position adjustment can be made in an efficient fashion, without requiring instrument detachment, thereby improving the surgical robot's efficiency and safety, shortening the time required for preoperative preparation, effectively reducing the risk and drawbacks associated with the conventional incision-making practice, increasing surgical accuracy, reducing patient trauma and pain and facilitating patient recovery.

Optionally, the first positional and postural information may include a distance and an angle of the support apparatus 400 with respect to the patient-side control device 200. It will be understood that a relative positional and postural relationship of the support apparatus 400 and the patient-side control device 200, i.e., the first positional and postural information, can be derived from said distance and angle.

Preferably, the locating unit 700 includes first and second terminals adapted to each other. The first terminal is configured to sense the position of the second terminal through a predetermined sensing medium. Depending on the type of the sensing medium, the first and second terminals may be provided on the patient-side control device 200 and/or the support apparatus 400.

Referring to Fig. 11, in an alternative embodiment, the sensing medium is light. The first terminal includes at least two optical photographing devices 711, and the second terminal includes an optical fiducial marker set 721 including at least three non-collinear optical fiducial markers 7210. The first terminal is disposed on the patient-side control device 200, and the second terminal is provided on the support apparatus 400. The optical photographing devices 711 may be, for example, NDI optical measurement devices, which may be mounted on a base 201 of the patient-side control device 200. The optical fiducial marker set 721 may be arranged on feature points of the support apparatus 400, for example. With the NDI optical measurement devices, the control unit 800 is able to determine a relative positional and postural relationship between the base 201 of the patient-side control device 200 and the support apparatus 400, and hence coordinates of the support apparatus centered at coordinates of the patient-side control device 200.

The optical photographing devices 711 utilize the binocular vision principles for detection. Reference is now made to Fig. 12a, a top view of the locating unit 700, and Fig. 12b, a front view of the locating unit 700, which show how location is achieved using the binocular vision principles. Since the two optical photographing devices 711 are fixedly mounted to the patient-side control device 200, the distance $a$ therebetween can be considered as a known value. The optical photographing devices 711 can measure angles *C* and *B* and distances *b* and *c*, from which distances from an optical fiducial marker 7210 to the optical photographing devices 711 can be obtained. Since the optical fiducial markers 7210 are fixedly mounted to the support apparatus 400, and because their coordinates are known, coordinates of plane in a coordinate system of the optical photographing devices 711 can be determined, given the fact that three non-collinear points can define a plane.

Additionally, the second terminal may include a plurality of optical fiducial marker sets 721 each disposed on one side of the support apparatus 400. Referring to Fig. 13, the support apparatus 400 may include a supporting board having four sides each mounted thereon with one optical fiducial marker set 721. Each optical fiducial marker set 721 includes N (≥3) scattered non-collinear optical fiducial markers 7210. In the illustrated embodiment, each optical fiducial marker set 721 includes four optical fiducial markers 7210. For each optical fiducial marker 7210, the control unit 800 can determine a distance and posture of the support apparatus 400 with respect to the optical fiducial marker 7210 from a measured distance from the patient-side control device 200 to the optical fiducial marker 7210.

Referring to Fig. 14, in a first example, one of the optical fiducial marker sets 721 is mounted at a respective one of the four sides of the supporting board of the support apparatus 400. Each optical fiducial marker set 721 may be an integrated set including N (≥3) non-collinear optical fiducial markers 7210, which operate in the same way as the aforementioned scattered optical fiducial markers 7210.

In a second example, the sensing medium is ultrasonic waves. The locating unit 700 includes an ultrasonic locating assembly. Reference is now made to Fig. 15a, a top view of the ultrasonic locating assembly, and Fig. 15b, a front view of the ultrasonic locating assembly. The first terminal includes at least two ultrasonic transmitters 712, and the second terminal includes at least two ultrasonic receivers 722. The first terminal is disposed on one of the support apparatus 400 and the patient-side control device 200, and the second terminal on the other.

The following description is set forth with reference to Fig. 16 in the exemplary context of the first terminal being provided on the support apparatus 400 and including two ultrasonic transmitters 712 and of the second terminal being provided on the patient-side control device 200 and including two ultrasonic receivers 722.

The two ultrasonic transmitters 712 are mounted on the support apparatus 400 and both communicatively connected to the control unit 800 (e.g., by wired or wireless connections). The ultrasonic transmitters 712 are configured to transmit ultrasonic waves under the control of the control unit 800. The two ultrasonic receivers 722 are mounted on the base 201 of the patient-side control device 200 and also both communicatively connected to the control unit 800. The ultrasonic receivers 722 can receive ultrasonic waves from the ultrasonic transmitters 712. The control unit 800 can calculate distances between the ultrasonic transmitters 712 and the ultrasonic receivers 722 from propagation times of ultrasonic waves between the ultrasonic transmitters 712 and the ultrasonic receivers 722. For example, if an ultrasonic wave is transmitted from one of the ultrasonic transmitters 712 at time t1 and received by one of the ultrasonic receivers 722 at time t2, then the distance s between the ultrasonic transmitter 712 and the ultrasonic receiver 722 can be calculated according to s=340* (t2-t1). Further, a defined distance and shape can be determined from distances from one of the ultrasonic transmitter 12 to the respective two ultrasonic receivers 722.

Further, each ultrasonic receiver 722 is configured to receive ultrasonic waves from each of the at least two ultrasonic transmitters 712, and the control unit 800 is further configured to be based on positional information of the robotic arm 210 in the patient-side control device 200 with respect to the support apparatus 400 to discard redundant positional information of the ultrasonic transmitters obtained from ultrasonic waves received at the ultrasonic receivers 722. With continued reference to Fig. 16, in fact, from an ultrasonic wave received by the two ultrasonic receivers 722 from a single ultrasonic transmitter 712, two solutions for the position of the ultrasonic transmitter 712 can be obtained, which are located respectively in front of (on the same side as the robotic arm 210) and behind (on the opposite side to the robotic arm 210) in axial symmetry with respect to a line connecting the ultrasonic receivers 722. One of the solutions is a redundant solution 7120, which can be identified based on the positional information of the robotic arm 210 with respect to the support apparatus 400, and the position of the ultrasonic transmitter 712 can be correctly determined. In this way, the relative positional and postural relationship of the support apparatus 400 and the patient-side control device 200 can be determined.

Referring to Fig. 17, in a third example, the sensing medium is a magnetic field. The first terminal includes a magnetic field generator 713, and the second terminal includes a magnetic locating sensor 723 (e.g., a magnetic locating coil, etc.) with at least three DoFs. The first terminal is disposed on the patient-side control device 200 and the second terminal on the support apparatus 400. For example, the magnetic field generator 713 may be attached to a surface of the base 201 in the patient-side control device 200, and the control unit 800 may determine coordinates of the support apparatus 400 in a coordinate system of the patient-side control device based on the collected information about the posture and the distance of the magnetic locating sensor 723.

In some embodiments, the magnetic locating sensor 723 is a coil with 3 DoFs, and the control unit 800 can determine X, Y and Z coordinates of the magnetic locating sensor 723 and derive its positional information from the coordinates. However, the control unit 800 cannot directly determine α, β and γ coordinates of the magnetic locating sensor 723 and therefore cannot obtain postural information of the magnetic locating sensor 723. Accordingly, the support apparatus 400 may optionally further include a gyroscope capable of collecting information of the $\alpha$, $\beta$ and $\gamma$ angular coordinates of the support apparatus 400. In this way, the control unit 800 can determine the position and posture of the support apparatus 400 with respect to the patient-side control device 200.

Preferably, the magnetic locating sensor 723 comprised in the second terminal has 6 DoFs, which can directly provide the X, Y, Z, $\alpha$, $\beta$ and $\gamma$ coordinates of the magnetic locating sensor 723 to the control unit 800 to allow it to determine the posture of the support apparatus 400. Of course, in some embodiments, the $\alpha$, $\beta$ and $\gamma$ coordinates obtained by the magnetic field generator 713 from the 6-DoF magnetic locating sensor 723 may be cross-checked with those collected by the gyroscope.

In a fourth example, the sensing medium is also a magnetic field. Reference is now made to Fig. 18a, a front view of another magnetic locating assembly, and Fig. 18b, perspective view of a magnetic locating sensor 723 mounted to the support apparatus 400. The magnetic field generator 713 is included in the first terminal, and the second terminal includes at least three non-collinear magnetic locating sensors 723. The first terminal is provided on the patient-side control device 200 and the second terminal on the support apparatus 400. In one exemplary implementation, the second terminal includes four magnetic locating sensors 723 disposed at the respective corners of the base of the support apparatus 400. With this arrangement, the control unit 800 can determine distances from the magnetic field generator 713 to the respective magnetic locating sensors 723 and derive therefrom the distance and angle of the support apparatus 400 with respect to the patient-side control device 200.

In a fifth example, the sensing medium is laser, and the locating unit 700 includes a laser locating assembly. Reference is now made to Fig. 19a, a top view of the laser locating assembly, Fig. 19b, a front view of the laser locating assembly, and Fig. 20. The first terminal includes a laser 714, and the second terminal includes a photographing device 724. Both the first and second terminals are disposed on the patient-side control device 200, or on the support apparatus 400. Both the laser 714 and the photographing device 724 can rotate within a predefined angular range. In one exemplary implementation, the laser 714 and the photographing device 724 are mounted on the base 201 of the patient-side control device 200. The support apparatus 400 can reflect laser radiation emitted from the laser 714. The laser 714 may emit pulsed laser radiation and rotate to scan the entire base of the support apparatus 400. The photographing device 724 may receive laser radiation. With this arrangement, the control unit 800 can computationally determine the distance between opposite ends of the base of the support apparatus 400 and derive therefrom the distance and posture of the support apparatus 400 with respect to the patient-side control device 200.

A measurement method based on laser reflection is described below with reference to Fig. 21. For a triangle with three sides a, b, c opposing vertices A, B, C, if an angle at the vertex B where the laser 714 is located and an angle at the vertex A where the photographing device 724 is located are both known, then an angle at the remaining vertex C is determinable. Since the laser 714 and the photographing device 724 are both fixed to the base 201 of the patient-side control device 200, the distance c therebetween can be considered as a known value. According to the law of sines, a/sinA=b/sinB=c/sinC, from which lengths of a and b can be calculated. In this way, the distance and angle of the support apparatus 400 with respect to the patient-side control device 200 can be determined.

Preferably, the locating unit 700 further includes a reflector plate 730 provided on the one of the patient-side control device 200 and the support apparatus 400, on which the laser 714 and the photographing device 724 are not disposed, and configured to reflect laser radiation emitted from the laser 714. For example, in one exemplary implementation, both the laser 714 and the photographing device 724 are provided on the patient-side control device 200, while the reflector plate 730 is disposed on the support apparatus 400.

Referring to Fig. 22, optionally, the system may further include a first position and posture acquisition unit 810 communicatively connected to the control unit 800 and configured to acquire second positional and postural information of the robotic arm 210 in the patient-side control device 200. The control unit 800 may derive information of coordinates of the RCM in the coordinate system of the patient-side control device from the second positional and postural information. For example, the first position and posture acquisition unit 810 may include encoders provided on joints of a telescopic upright, a suspension, an adjustment arm or an instrument arm in the patient-side control device 200. Each encoder can feed back information about a translation distance or rotation angle of a motor for a respective one of the joints. From feedback information from the encoders, the control unit 800 can derive information about the coordinates of the RCM in the coordinate system of the patient-side control device. Specifically, assuming that there are N joints, each of which is mounted thereon with an encoder for translation distance and rotation angle calculation, between the patient-side control device 200 with coordinates denoted as ${}^{0}_{1}T$ and the RCM with coordinates denoted as ${}^{0}_{N}T$, the coordinates of the terminal RCM can be simply calculated using DH parameters according to:

$$ {}^{0}_{N}T = {}^{0}_{1}T\, {}^{1}_{2}T\, {}^{2}_{3}T\, ...\, {}^{N-1}_{N}T. $$

Referring to Fig. 23, optionally, the system may further include a second position and posture acquisition unit 820 (which may include an encoder provided on a telescopic upright 821 and a gyroscope provided on a rotary joint 822) communicatively connected to the control unit 800 and configured to acquire third positional and postural information of the supporting board of the support apparatus 400. From the third positional and postural information, the control unit may derive information of coordinates of the supporting board in a coordinate system of the support apparatus. Generally, the support apparatus 400 includes a telescopic upright 821 and a rotary joint 822, which are configured to enable lifting/lowering and rotation of the supporting board, respectively. The third positional and postural information may include information about a lifted/lowered height and rotation angle of the supporting board. The supporting board can rotate in all directions about the rotary joint 822, as shown in Fig. 23. Preferably, the second position and posture acquisition unit 820 includes an encoder provided on the telescopic upright and a gyroscope provided on the rotary joint. The encoder on the telescopic upright can feed back information about a lifted/lowered height of the supporting board, and the gyroscope on the rotary joint can feed back information about a rotation angle of the supporting board. Thus, the coordinates of the supporting board in the coordinate system of the support apparatus can be simply determined using DH parameters

Referring to Fig. 24, optionally, the patient-side control device 200 and the support apparatus 400 may be placed in the same horizontal plane and thus have the same z coordinate. Thus, coordinates of the coordinate system of the support apparatus in the coordinate system of the patient-side control device can be simply derived from the distance (x coordinate) and angle (y coordinate) of the support apparatus 400 with respect to the patient-side control device 200.

On the basis of the system as discussed above, in one embodiment of the present invention, there is also provided a surgical robot system comprising a support apparatus 400, a patient-side control device 200 and the RCM follow-up adjustment system for support apparatus as defined above. The RCM follow-up adjustment system for support apparatus is used to control movement of the patient-side control device 200 and thereby maintain an RCM positionally and posturally unchanged with respect to the support apparatus 400.

An intraoperative RCM adjustment method provided herein will be described below with reference to the following drawings.

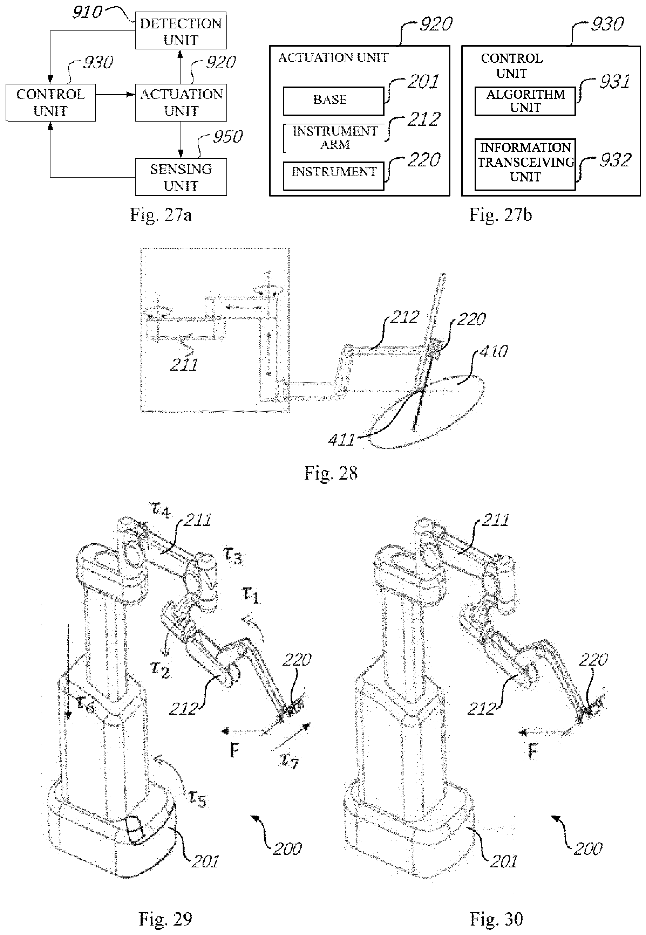

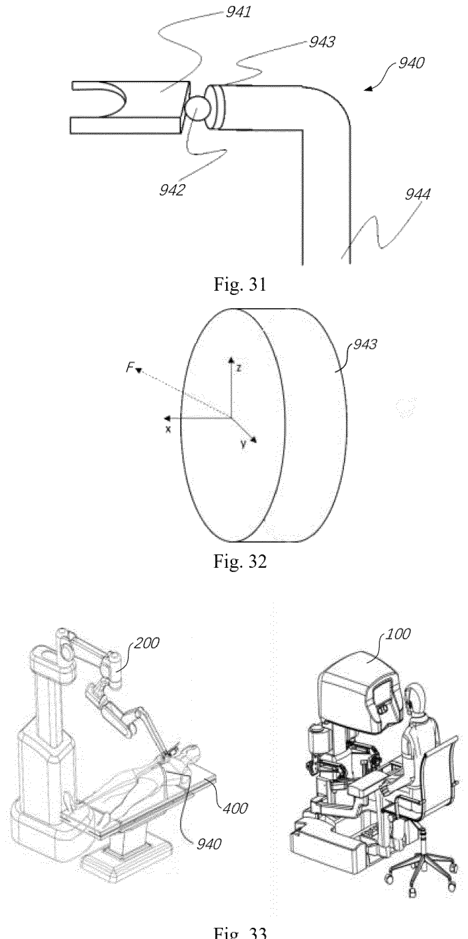

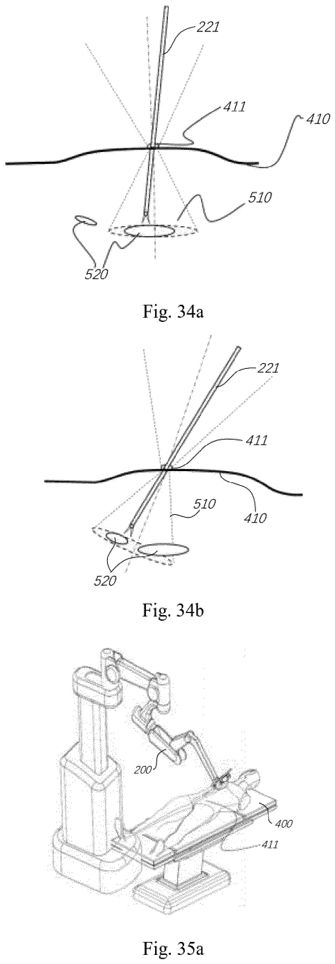

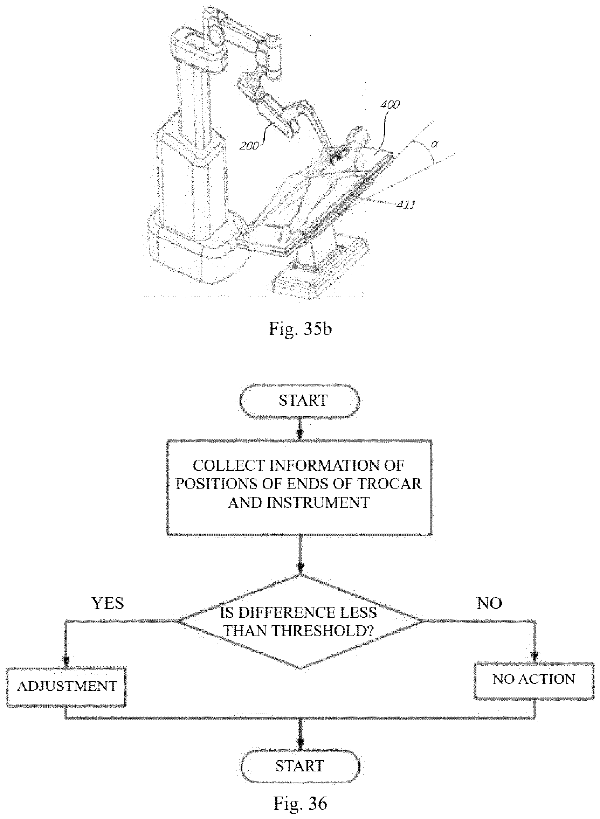

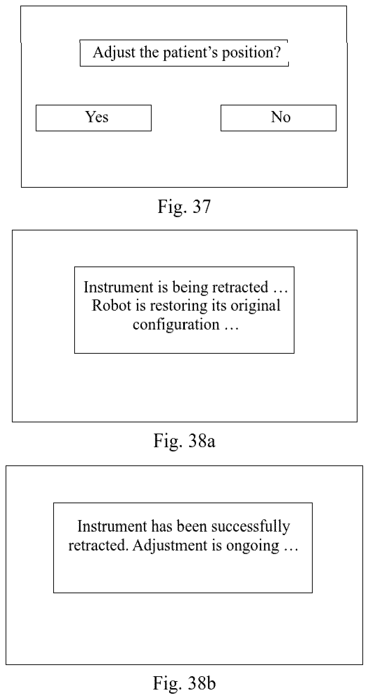

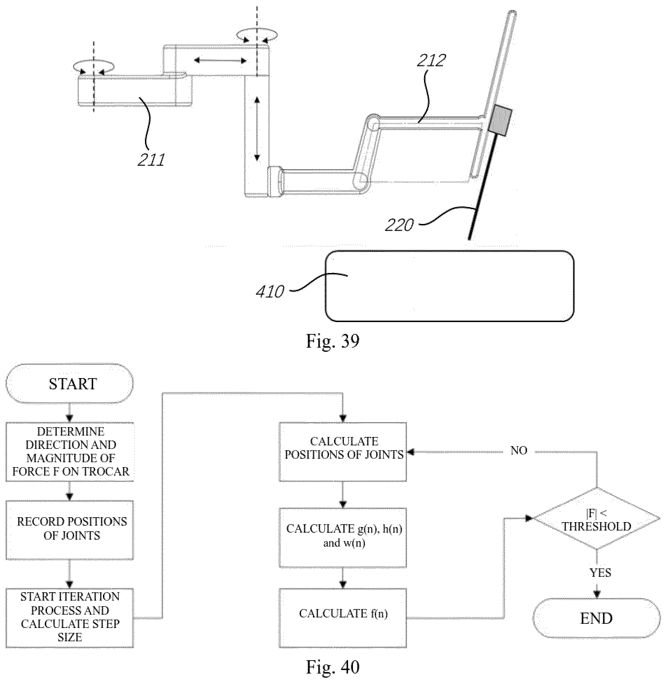

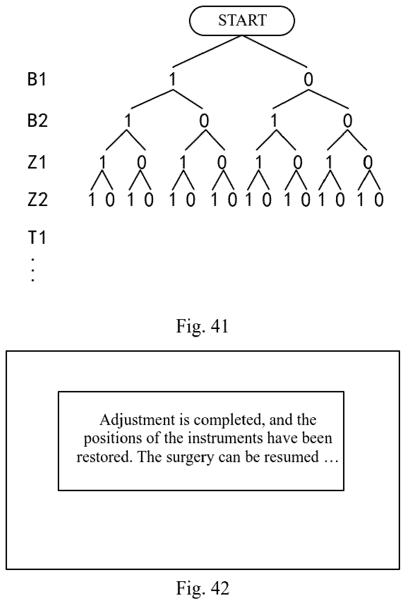

Reference will be made to Figs. 25 to 42 below. Fig. 25 is a flowchart of an intraoperative remote center of motion adjustment method according to an embodiment of the present application. Fig. 26 schematically depicts an instrument inserted through a trocar according to an embodiment of the present application. Figs. 27a and 27b schematically depict an intraoperative RCM adjustment system according to an embodiment of the present application. Fig. 28 schematically depicts a mechanism RCM according to an embodiment of the present application. Fig. 29 schematically depicts deriving a force acting on an instrument from torques on joints according to an embodiment of the present application. Fig. 30 schematically depicts determining a force acting on an instrument using a torque sensor according to an embodiment of the present application. Fig. 31 schematically depicts a trocar fixation assembly according to an embodiment of the present application. Fig. 32 is a diagram showing mechanic analysis of a trocar according to an embodiment of the present application. Fig. 33 schematically depicts determining a force acting on a trocar with a 3D force sensor according to an embodiment of the present application. Figs. 34a and 34b schematically depict an operating space for an instrument before and after it is adjusted according to an embodiment of the present application. Figs. 35a and 35b schematically depict a patient before and after his/her position is adjusted according to an embodiment of the present application. Fig. 36 schematically depicts an adjustment confirmation step according to an embodiment of the present application. Fig. 37 schematically depicts an indication displayed for confirmation of adjustment according to an embodiment of the present application. Figs. 38a and 38b schematically depict indications provided in an adjustment process according to an embodiment of the present application. Fig. 39 schematically depicts an instrument retracted back into a trocar according to an embodiment of the present application. Fig. 40 schematically depicts an iterative algorithm according to an embodiment of the present application. Fig. 41 schematically depicts rules of movements for joints according to an embodiment of the present application. Fig. 42 schematically depicts a displayed indication indicative of the completion of adjustment according to an embodiment of the present application.

As shown in Fig. 25, according to one embodiment, the method includes the steps as follows:

Step SC1: Determine an external force. During movement of an instrument 220 attached to a robotic arm 210 of a patient-side control device 200 relative to a guide tube disposed around the instrument 220, a force acting on the instrument 220 and/or a force acting on the guide tube is/are determined. The instrument 220 attached to the robotic arm 210 is configured to move while passing through an RCM. It is to be noted that, here, by "a guide tube disposed around the instrument 220", it is meant to mean that the guide tube surrounds at least part of the instrument 220 (i.e., across part of its axial length) rather than necessarily the entirety of the instrument 220 (i.e., across its entire axial length).

Step SC2: Make adjustment. Based on the determined force on the instrument 220 and/or force on the guide tube, a position and posture of the robotic arm 210 is adjusted so as to maintain the RCM positionally and posturally unchanged with respect to a support apparatus 400.

With this arrangement, intraoperative RCM adjustment can be made so that the position and posture of the robotic arm 210 is adjusted according to the determined force on the instrument 220 and/or force on the guide tube. This enables real-time adjustment of the robotic arm 210 according to the adjustment of RCM, ensuring that the RCM remains positionally and posturally unchanged with respect to the support apparatus 400. The intraoperative position adjustment can be made without interrupting the ongoing surgical procedure, addressing a limited space for movement of robotic arms, non-ideal incision positions and other problems associated with the prior art, which arises from a robot/patient positional relationship. Moreover, various forms of intraoperative position adjustment can be made in an efficient fashion, without requiring instrument detachment, thereby improving the surgical robot's efficiency and safety, shortening the time required for preoperative preparation, effectively reducing the risk and drawbacks associated with the conventional incision-making practice, increasing surgical accuracy, reducing patient trauma and pain and facilitating patient recovery.

The intraoperative RCM adjustment method according to this embodiment is mainly used to impart real-time servo adjustment to one of the robotic arm 210 and the support apparatus 400 as a lead object of adjustment and the other as a follower object of adjustment. As used herein, the term "guide tube" principally refers to a protective stopper disposed through an incision 411 made in the body surface of a patient, which can limit and guide the instrument 220 inserted into the body of the patient. Although the guide tube is exemplified below as a trocar 900, it would be appreciated that the trocar 900 is only an example of the guide tube and not intended to limit the scope of the invention. Those skilled in the art may also select other components as the guide tube. Referring to Fig. 26, in one application scenario of the surgical robot system of the invention, after the incisions are made in the patient in step SO2, each incision 411 is inserted therethrough with a trocar 900, through which the instrument 220 attached to a respective one of the robotic arms 210 is in turn inserted into the body of the patient. It will be understood that, as a result of this, a mechanical RCM is defined at an intersection of an axis of the trocar 900 with the patient's body surface. During intraoperative adjustment made to the robotic arm 210 or the support apparatus 400, the instrument 220 may move relative to the trocar 900 and come into contact therewith, resulting in exertion of forces by the instruments 220 and the trocar 900 on each other.

The force acting on at least one of the instrument 220 and the trocar 900 may be determined, and based on which, an adjustment path may be calculated for the robotic arm 210. In this way, following any adjustment to the RCM, the position and posture of the robotic arm 210 can be correspondingly adjusted in real time to reduce compression of the trocar 900 on the incision and hence damage caused to the patient.

Fig. 27a schematically depicts an intraoperative RCM adjustment system for implementing the intraoperative RCM adjustment method according to the present embodiment. As shown, the intraoperative RCM adjustment system includes a sensing unit 950, an actuation unit 920 and a control unit 930. The actuation unit 920 includes a robotic arm 210 configured for attachment thereto of an instrument 220 inserted through a guide tube. The sensing unit 950 is used to determine a force acting on the instrument 220 and/or a force acting on the guide tube. The control unit 930 is communicatively connected to both the sensing unit 950 and the actuation unit 920 and configured to control positional and postural adjustment of the robotic arm 210 according to the above-described intraoperative RCM adjustment method so as to maintain an RCM positionally and posturally unchanged with respect to a support apparatus 400. The sensing unit 950 may include a torque sensor disposed on a base 201 of a patient-side control device 200, a force sensor 943 disposed on a trocar 900, or the like.

Preferably, the system further includes a detection unit 910 communicatively connected to the control unit 930 and configured to detect postural variation information of the actuation unit 920. The control unit 930 is configured to provide closed-loop control over the actuation unit 920 and the detection unit 910. In one example, when receiving the positional and postural variation information of the actuation unit 920 detected by the detection unit 910, the control unit 930 processes it using a predefined algorithm to derive action information (e.g., containing commands for joint motors) therefrom and then provides the action information to the actuation unit 920. As an effector mechanism, the actuation unit 920 takes actions indicated in the action information from the control unit 930, and the detection unit 910 in turn detects positional and postural variation of the actuation unit 920 occurring in this process, thereby achieving closed-loop control. Referring to Fig. 27b, in one example, the actuation unit 920 includes a robotic arm 210 and an instrument 220 both of the patient-side control device 200. In a more particular embodiment, the actuation unit 920 includes a base 201, an instrument arm 212 and an instrument 220, and the control unit 930 includes an algorithm unit 931 (containing the contents of the algorithm (for processing received information) and a CPU) and an information transceiving unit 932 (for receiving and transmitting information). For example, the detection unit 910 may include a position sensor 610 (e.g., a binocular vision apparatus), a fiducial marker 620, etc. and can detect positional and postural variation information of the robotic arm 210 in real time.

Referring to Fig. 28, in an exemplary embodiment, an adjustment arm 211, a mechanism RCM and an instrument 220 are included. The mechanism RCM is implemented as an incision made in a patient's body surface, and when not in an adjustment process performed by the adjustment arm 211, adjustment to any other joint has no impact on the position and posture of the mechanism RCM. A few examples are set forth below to explain how a force acting on the instrument 220 and/or an force acting on a trocar 900 is/are determined.

Referring to Fig. 29, in a first example, the force acting on the instrument 220 is detected using dynamic equations. This may include:

step SC11, determining torques on joints in the patient-side control device 200; and
step SC12, deriving the force acting on the instrument 220 from the torques on the joints.

According to robot dynamic equations, each of the external torques can be determined according to the following formula:

$$\tau_{ext} = M(q)\ddot{q} + C(q, \dot{q}) + g(q) - \tau_{m},$$

where $\tau_{ext}$ is the external torque, $q$ is the position of the joint, $\dot{q}$ is a moving speed of the joint, $\ddot{q}$ is an acceleration of the joint, $\tau_{m}$ is an output torque of the motor, and M($q$), C($q, \dot{q}$) and g($q$) represent inertia force, Coriolis force and gravity, respectively. The external torques on the joints may be combined to derive the magnitude and direction of the external force acting on the instrument 220. The external torques on the joints may be detected using torque sensors disposed on the joints, for example. The combination of the external torques on the joints and the derivation of the magnitude and direction of the external force F may be accomplished as is conventional, and further description thereof is omitted herein.

Referring to Fig. 30, in a second example, the magnitude and direction of the external force F acting on the instrument 220 are determined using a torque sensor provided on the base 201 of the patient-side control device 200 according to a method including:

step SC13, determining an external force exerted on the robotic arm 210 from a force on the base 201 of the patient-side control device 200 detected by the torque sensor; and
step SC14, deriving the force acting on the instrument 220 from the external force on the robotic arm 210. Optionally, the torque sensor disposed on the base 201 may be a six-axis force/torque sensor. The external force on the robotic arm 210 can be calculated from output torque information from the six-axis force/torque sensor, and the magnitude and direction of the external force F acting on the instrument 220 arranged on an end of the robotic arm 210 can be in turn derived from the external force on the robotic arm 210. This can be accomplished as is conventional, and further description thereof is omitted herein.

Referring to Figs. 31 and 33, in a third example, the external force acting on the trocar 900 is determined using a trocar fixation assembly 940 including a fixation member 941, a passive joint 942, a force sensor 943 and an attachment member 944, which are disposed and connected sequentially between the trocar 900 and the support apparatus 400. The fixation member 941 is configured to retain the trocar 900 without movement relative thereto. The passive joint 942 is configured to ensure that motion of the trocar 900 is decoupled from the support apparatus 400 and thus will not be affected by any other component located proximally with respect to the passive joint 942. For example, the force sensor 943 may be a 3D force sensor configured to measure a force acting on the trocar 900 in a 3D space. The attachment member 944 is attached to the support apparatus 400. Fig. 32 shows the force acting on the trocar 900. If without the fixation member 941 and the attachment member 944, the trocar 900 would experience positional variation in response to any adjustment made to the support apparatus 400 for changing the position of the patient. The presence of the fixation member 941 and the attachment member 944 can ensure the trocar 900 is kept stationary relative to the support apparatus 400. In response to any movement of the support apparatus 400, the force sensor 943 may produce a force signal indicating a force in a 3D space, which may be synthesized into a spatial force vector representative of the force acting on the trocar 900.

Accordingly, the determination of the force acting on the trocar 900 may include:

step SC15, acquiring a force signal detected by the force sensor 943 disposed on the trocar 900; and
step SC16, obtaining the force acting on the trocar 900 from the force signal.

Figs. 34a and 34b schematically illustrate operating spaces respectively before and after adjustment is made to the instrument 220. Fig. 34a shows an operating space 510 before the instrument 220 is adjusted. As can be seen, the operating space 510 is conical. If this operating space 510 for the instrument 220 does not fully encompass a lesion area 520, the robotic arm 210 (essentially, the instrument arm 212) may be moved to expand the operating space for the instrument 220 so that the adjusted operating space 510 can encompass the entire lesion area 520, as shown in Fig. 34b.

Figs. 35a and 35b illustrate how an operator changes the position of the patient by adjusting the support apparatus 400. In one example, the support apparatus 400 is tilted by an angle of α so that the lesion therein is present within the current operating space of the instrument 220. Since this would alter the position of the RCM with respect to the support apparatus 400, it is necessary to adjust the robotic arm 210 at the same time to keep the RCM positionally unchanged with respect to the support apparatus 400.

Optionally, referring to Fig. 36, the intraoperative RCM adjustment method may include an adjustment confirmation step SC0, in which an indication of whether the position of the patient is to be adjusted is provided for the operator's confirmation. When receiving information indicating that the operator has confirmed the adjustment to be made, the control unit 930 may first collect information of positions of ends of the trocars 900 and the respective instruments 220 and determine whether the difference between the two positions is less than a preset threshold. If this is true, then the patient's position will be adjusted only when it has been confirmed that the instrument 220 is retracted back into the trocar 900. If it is not the case, the patient's position will not be adjusted to avoid damage caused to the patient during the adjustment of the patient's position. The indication may be provided in various forms. For example, it may be presented on an imaging device 102 or a display device 302, as shown in Fig. 37. Alternatively, it may also be provided in the form of a graphic button, sound, light, etc. The present invention is not limited to any particular form in which the indication is provided.

In addition, referring to Figs. 38a and 38b, after confirming that the instrument 220 has been retracted into the trocar 900, the control unit 930 controls the robotic arm 210 and the support apparatus 400 to effect the adjustment. In this process, indications may be provided to the operator, like "Instrument is being retracted ... Robot is restoring its original configuration ..." and "Instrument has been successfully retracted. Adjustment is ongoing ..." Moreover, in this process, the operator cannot take any control action on the patient-side control device 200, and the latter remains in the position before the process begins. The indications may be provided in various forms. For example, it may be presented on the imaging device 102 or the display device 302. Alternatively, it may also be provided in the form of a graphic button, sound, light, etc. The present invention is not limited to any particular form in which the indications are provided.

Further, referring to Fig. 39, after the instrument 220 is retracted into the trocar 900 and thus completely withdrawn from the patient's body, the robotic arm 210 may be restored to a predetermined position using inverse kinematics, thereby ensuring no damage caused to the patient during the operating space adjustment.

Preferably, the position and posture of the robotic arm 210 may be adjusted based on the force on the trocar 900 in a numerical or analytical manner, or using robot kinematics. Before the position and posture of the robotic arm 210 is adjusted, it is necessary to determine an adjustment path for the robotic arm 210. Likewise, this may also be accomplished in a numerical or analytical manner, or using robot kinematics (such as the Jacobi method).

Referring to Fig. 40, adjusting the position and posture of the robotic arm 210 in a numerical manner may include:

step SC21, acquiring positional and postural information of the RCM and positional information of the joints of the robotic arm 210;
step SC22, based on the positional and postural information of the RCM and the positional information of the joints of the robotic arm 210, calculating all possible adjustment paths for the robotic arm 210, according to a predefined algorithm;
step SC23, screening the possible adjustment paths for a desired adjustment path for the robotic arm 210.

The predefined algorithm may include:
for each joint, from the magnitude of the force F acting on the trocar 900, a range $P_r$ of movement across the entire travel of the joint, a step count *counts* of the motor associated with the joint, and a threshold *value* for step size amplification, an iterative step size $S$ is calculated for the joint according to:

$$S = \frac{P_r}{counts} * \frac{F}{value}.$$

For each joint, after the iterative step size $S$ is obtained, its displacement increment is calculated, which is defined as a distance that the joint moves in each step. Such movement follows rules as shown in Fig. 41, in which for each of motors B1, B2 on the base 201, motors Z1, Z2 on a suspension and motors T1, T2, T3, T4, ... on the adjustment arm 211, its forward rotation is labeled as "1" and backward rotation as "0". In this way, all the possible adjustment paths for the robotic arm 210 can be obtained.

The screening for the desired adjustment path for the robotic arm 210 in step SC23 may include:
step SC231, subjecting one or more of the possible adjustment paths for the robotic arm 210 to calculation using a convergence determining function f(n).

Preferably, the convergence determining function f(n) is subject to convergence conditions and constraint conditions.

The convergence conditions may include: coincidence of a direction of movement of an end of the robotic arm 210 with the direction of the force F acting on the trocar 900; and no increase in a force exerted on the end of the robotic arm 210.

The constraint conditions may include at least one of the following conditions.

1) The robotic arm 210 does not collide with any other robotic arm 210. For example, for any two robotic arms 210, this means the following equation is satisfied:

$$\iota = \frac{|(\vec{I_1} \times \vec{I_2}) \cdot \overrightarrow{PQ}|}{|\vec{I_1} \times \vec{I_2}|}$$

where $\iota$ is the distance between the two robotic arms 210, $\vec{I_1}$ is a unit vector of a straight line along which one of the robotic arms extends, $\vec{I_2}$ is a unit vector of a straight line along which the other of the robotic arms extends, and $\overrightarrow{PQ}$ is a vector of a line connecting motor boxes on instruments attached to the respective two robotic arms 210.

2) a positional and postural change of the end of the robotic arm 210 is less than a predetermined threshold, i.e., $Pose_c$-$Pose_p$ < threshold,

where $Pose_c$ and $Pose_p$ represent current and initial positions and postures of the end of the robotic arm, and threshold denotes the predetermined threshold for positional and postural change of the end of the robotic arm 210.

3) The position of each joint is within a predetermined position range, i.e.,

$$P_{min} + 20\% * P_r < P_c < P_{max} - 20\% * P_r,$$

where $P_{min}$ and $P_{max}$ represent minimum and maximum position limits, respectively, and $P_r$ and $P_c$ are a range of movement and the current position of the joint, respectively.

The convergence determining function f(n) is used to determine whether the magnitude of the force F on the trocar 900 is less than a threshold. If not, the positions of the joints are recalculated, and a new path is determined and verified to see whether it meets the above criterion. If so, commands are sent to the joint motors to instruct them to take actions indicated in the commands.

The screening for the desired adjustment path for the robotic arm 210 in step SC23 may further include:

step SC232, subjecting all the possible adjustment paths for the robotic arm 210 to calculation using a cost function g(n), a heuristic function h(n) and a weighting function w(n). All the cost function g(n), the heuristic function h(n) and the weighting function w(n) may be known functions in the art. An example of the calculation using the cost function g(n), the heuristic function h(n) and the weighting function w(n) is given below.

The cost function g(n) may be expressed as:

$$g(n) = \sum_{i=1}^{n} 0.3 * \alpha_i + \sum_{i=1}^{n} 0.3 * \beta_i + \sum_{i=1}^{n} 0.2 * \gamma_i + \sum_{i=1}^{n} 0.2 * \delta_i,$$

where $\alpha_i = \sum_{j=1}^{3} \frac{1}{l_j}$ , and $l_j$ = distance(Arm&Arm) representing the distance between non-coplanar straight lines.

$$\beta_i = \sum_{j=1}^{4} \lambda_j * \frac{1}{\eta_\beta}$$

Moreover, , and $\lambda_j$ = $Point_c$ - $Point_p$, where $Point_c$ and $Point_p$ represent current and initial postures of the RCM, respectively.

$$\gamma_i = \sum_{j=1}^{4} \mu_j * \frac{1}{\eta_\gamma}$$

Additionally, , and $\mu_j$ = $Pose_c$ - $Pose_p$, where $Pose_c$ and $Pose_p$ are the current and initial postures of the end of the robotic arm.

$$\delta_i = \sum_{j=1}^{m} v_j * \frac{1}{\eta_\delta}$$

Further, $\hspace{3cm}$ , and $v_j = Pos_c\text{-}Pos_p$, where m is the number of motors, and this value may vary with the presence or absence of the adjustment arm 211 in the robotic arm.

Furthermore, $\eta_\beta$, $\eta_\gamma$, $\eta_\delta$ are scaling factors, which are 0 when $\lambda_j$, $\mu_j$, $\eta_\delta$ are above a threshold, or are 1 when $\lambda_j$, $\mu_j$, $\eta_\delta$ are below the threshold.

The heuristic function h(n) may be expressed as:

$$h(n) = abs(\theta_c - \theta_t),$$

where $\theta_c$ represents a current position of a limited joint; and
$\theta_t$ is a target position for the limited joint (e.g., the midpoint of the entire travel).

$$f(n) = g(n) + \omega(n) * h(n),$$

$\omega(n)$ is a weight of the heuristic function, and $\omega \geq 1$. When approaching the target, the weight is reduced to avoid collision between robotic arms and maintain the RCM positionally and posturally unchanged with respect to an end of the instrument during adjustment following the path of interest.

Optionally, referring to Fig. 42, after the adjustment process is completed, an indication of this may be provided to the operator, like "Adjustment is completed, and the positions of the instruments have been restored. The surgery can be resumed ..." Likewise, the present invention is not limited to any particular form of the indication.

Furthermore, following any positional and postural adjustment made to the robotic arm 210, the robot may also perform the same self-adaptive adjustment as in step SO6 described above to tune itself to a desirable position and posture.

In summary, the present application provides an intraoperative RCM adjustment method, a readable storage medium and a surgical robot system. The method includes: during movement of an instrument attached to a robotic arm in a patient-side control device relative to a guide tube disposed around the instrument, determining a force acting on the instrument and/or a force acting on the guide tube, wherein the instrument attached to the robotic arm is configured to move while passing through an RCM; and according to the determined force on the instrument and/or force on the guide tube, adjusting a position and posture of the robotic arm so that the RCM remains positionally and posturally unchanged with respect to a support apparatus.

With this arrangement, intraoperative RCM adjustment can be made so that the position and posture of the robotic arm is adjusted according to the determined force on the instrument and/or force on the guide tube. This enables real-time adjustment of the robotic arm according to the adjustment of RCM, ensuring that the RCM remains positionally and posturally unchanged with respect to the support apparatus. The intraoperative position adjustment can be made without interrupting the ongoing surgical procedure, addressing a limited space for movement of robotic arms, non-ideal incision positions and other problems associated with the prior art, which arises from a robot/patient positional relationship. Moreover, various forms of intraoperative position adjustment can be made in an efficient fashion, without requiring instrument detachment, thereby improving the surgical robot's efficiency and safety, shortening the time required for preoperative preparation, effectively reducing the risk and drawbacks associated with the conventional incision-making practice, increasing surgical accuracy, reducing patient trauma and pain and facilitating patient recovery.

It is to be noted that the foregoing several embodiments are not limited to being used alone but can be combined. The present invention is not limited in this regard. The description presented above is merely that of a few preferred embodiments of the present invention and does not limit the scope thereof in any sense. Any and all changes and modifications made by those of ordinary skill in the art based on the above teachings are intended to fall within the scope of the present invention.

## Claims

1. A remote center of motion follow-up adjustment system for support apparatus, comprising a locating unit and a control unit,

the control unit communicatively connected to the locating unit, the control unit configured to acquire, through the locating unit, first positional and postural information of a support apparatus with respect to a patient-side control device,

the control unit configured to monitor, based on the first positional and postural information, positional and postural variation of the support apparatus with respect to the patient-side control device, wherein a robotic arm in the patient-side control device is configured to drive an instrument attached thereto to move while passing through an RCM,

the control unit configured to, when the positional and postural variation of the support apparatus with respect to the patient-side control device is identified to exceed a predetermined value, control the patient-side control device to move so that the RCM remains unchanged in terms of position and posture with respect to the support apparatus.

2. The remote center of motion follow-up adjustment system for support apparatus according to claim 1, wherein the first positional and postural information comprises a distance of the support apparatus with respect to the patient-side control device and an angle of the support apparatus with respect to the patient-side control device.

3. The remote center of motion follow-up adjustment system for support apparatus according to claim 1, further comprising a first position and posture acquisition unit communicatively connected to the control unit, the first position and posture acquisition unit configured to acquire second positional and postural information of the robotic arm in the patient-side control device, wherein the control unit derives information of coordinates of a remote center of motion in a coordinate system of the patient-side control device from the second positional and postural information.

4. The remote center of motion follow-up adjustment system for support apparatus according to claim 1, further comprising a second position and posture acquisition unit communicatively connected to the control unit, the second position and posture acquisition unit configured to acquire third positional and postural information of a supporting board in the support apparatus, wherein the control unit derives information of coordinates of the supporting board in a coordinate system of the support apparatus from the third positional and postural information.

5. The remote center of motion follow-up adjustment system for support apparatus according to claim 4, wherein the third positional and postural information comprises information of a lifted/lowered height of the supporting board and a rotation angle of the supporting board, wherein the second position and posture acquisition unit comprises an encoder disposed on a telescopic upright and a gyroscope disposed on a rotary joint, the encoder configured to feed back the information of the lifted/lowered height of the supporting board, the gyroscope configured to feed back the rotation angle of the supporting board.

6. The remote center of motion follow-up adjustment system for support apparatus according to claim 1, wherein the locating unit comprises first and second terminals adapted to each other, the first terminal configured to sense a position of the second terminal through a predetermined sensing medium, the first and second terminals disposed on the patient-side control device and/or on the support apparatus depending on a type of the sensing medium and configured to acquire the first positional and postural information of the support apparatus with respect to the patient-side control device.

7. The remote center of motion follow-up adjustment system for support apparatus according to claim 6, wherein the first terminal comprises at least two optical photographing devices, wherein the second terminal comprises an optical fiducial marker set comprising at least three non-collinear optical fiducial markers, and wherein the first terminal is disposed on the patient-side control device, and the second terminal is disposed on the support apparatus.

8. The remote center of motion follow-up adjustment system for support apparatus according to claim 7, wherein the second terminal comprises a plurality of optical fiducial marker sets each disposed a side edge of the support apparatus.

9. The remote center of motion follow-up adjustment system for support apparatus according to claim 6, wherein the first terminal comprises at least two ultrasonic transmitters, wherein the second terminal comprises at least two ultrasonic receivers, and wherein the first terminal is disposed on one of the support apparatus and the patient-side control device, and the second terminal is disposed on the other of the support apparatus and the patient-side control device.

10. The remote center of motion follow-up adjustment system for support apparatus according to claim 9, wherein each of the ultrasonic receivers is configured to receive ultrasonic waves transmitted from at least two of the ultrasonic transmitters, wherein the control unit is also configured to discard, based on positional information of the robotic arm in the patient-side control device with respect to the support apparatus, redundant positional information of the ultrasonic transmitters obtained based on the ultrasonic receivers.

11. The remote center of motion follow-up adjustment system for support apparatus according to claim 6, wherein the first terminal comprises a magnetic field generator, wherein the second terminal comprises a magnetic locating sensor having at least 3 degrees of freedom, and wherein the first terminal is disposed on the patient-side control device, and the second terminal is disposed on the support apparatus.

12. The remote center of motion follow-up adjustment system for support apparatus according to claim 11, wherein the second terminal comprises a magnetic locating sensor having at least 6 degrees of freedom.

13. The remote center of motion follow-up adjustment system for support apparatus according to claim 6, wherein the first terminal comprises a magnetic field generator, wherein the second terminal comprises at least three non-collinear magnetic locating sensors, and wherein the first terminal is disposed on the patient-side control device, and the second terminal is disposed on the support apparatus.

14. The remote center of motion follow-up adjustment system for support apparatus according to claim 6, wherein the first terminal comprises a laser, wherein the second terminal comprises a photographing device, and wherein both the first and second terminals are disposed on the patient-side control device, or both the first and second terminals are disposed on the support apparatus.

15. The remote center of motion follow-up adjustment system for support apparatus according to claim 14, wherein the locating unit further comprises a reflector plate disposed on the one of the patient-side control device and the support apparatus, on which the laser and the photographing device are not disposed, and configured to reflect laser radiation emitted from the laser.

16. A surgical robot system, comprising a support apparatus, a patient-side control device and the remote center of motion follow-up adjustment system for support apparatus according to any of claims 1 to 15, wherein the remote center of motion follow-up adjustment system for support apparatus is configured to control the patient-side control device to move so that a remote center of motion remains unchanged in terms of position and posture with respect to the support apparatus.

17. An intraoperative remote center of motion adjustment method, comprising:

during movement of an instrument attached to a robotic arm in a patient-side control device relative to a guide tube disposed around the instrument, determining a force acting on the instrument and/or a force acting on the guide tube, wherein the instrument attached to the robotic arm is configured to move while passing through an RCM; and
according to the determined force on the instrument and/or force on the guide tube, adjusting a position and posture of the robotic arm so that the RCM remains unchanged in terms of position and posture with respect to a support apparatus.

18. The intraoperative remote center of motion adjustment method according to claim 17, wherein determining the force acting on the instrument comprises:

determining torques on joints in the patient-side control device; and
deriving the force acting on the instrument from the torques on the joints.

19. The intraoperative remote center of motion adjustment method according to claim 17, wherein determining the force acting on the instrument comprises:

acquiring a force detected by a torque sensor disposed on a base of the patient-side control device to derive an external force acting on the robotic arm; and
deriving the force acting on the instrument from the external force acting on the robotic arm.

20. The intraoperative remote center of motion adjustment method according to claim 17, wherein determining the force acting on the guide tube comprises:

> acquiring a force signal detected by a force sensor disposed on the guide tube; and
> obtaining the force acting on the guide tube based on the force signal.

21. The intraoperative remote center of motion adjustment method according to claim 17, wherein adjusting the position and posture of the robotic arm according to the determined force on the guide tube comprises:
according to the force on the guide tube, adjusting the position and posture of the robotic arm numerically, analytically, or using robot kinematics.

22. The intraoperative remote center of motion adjustment method according to claim 21, wherein adjusting the position and posture of the robotic arm numerically comprises:

> acquiring position and postural information of the RCM and positional information of joints in the robotic arm;
> based on the postural information of the RCM and the positional information of the joints, calculating a plurality of adjustment paths for the robotic arm according to a predefined algorithm; and
> screening for a desired one of the adjustment paths for the robotic arm.

23. The intraoperative remote center of motion adjustment method according to claim 22, wherein the predefined algorithm comprises:
for each joint, from a magnitude of the force acting on the guide tube, a range of movement across an entire travel of the joint, a step count of a motor associated with the joint and a threshold for step size amplification, an iterative step size is calculated for the joint; and thereby deriving all possible adjustment paths for the robotic arm.

24. The intraoperative remote center of motion adjustment method according to claim 23, wherein screening for the desired adjustment path for the robotic arm comprises:
subjecting one or more of the possible adjustment paths for the robotic arm to calculation using a convergence determining function.

25. The intraoperative remote center of motion adjustment method according to claim 24, wherein the convergence determining function is subject to conditions comprising convergence conditions and constraint conditions,

> the convergence conditions comprising coincidence of a moving direction of an end of the robotic arm with a direction of the force acting on the guide tube and no increase in a force acting on the end of the robotic arm,
> the constraint conditions comprising at least one of:
> no collision between robotic arms, a positional and postural change of the end of the robotic arm less than a predetermined threshold, and positions of the joints lying within a predetermined position range.

26. The intraoperative remote center of motion adjustment method according to claim 24, wherein screening for the desired adjustment path for the robotic arm further comprises:
subjecting all the possible adjustment paths for the robotic arm to calculation using a cost function, a heuristic function and weighting function.

27. The intraoperative remote center of motion adjustment method according to claim 17, further comprising, after the position and posture of the robotic arm is adjusted according to the determined force on the instrument and/or force on the guide tube,

> according to the adjusted position and posture of the robotic arm, adjusting the instrument attached to the robotic arm to a desired position and posture; and
> synchronizing a position and posture of a control manipulator in a surgeon's control terminal to the position and posture of the robotic arm.

28. A readable storage medium, storing thereon a program, wherein the program, when executed, implements the intraoperative remote center of motion adjustment method according to any one of claims 17 to 27.

29. A remote center of motion adjustment system, comprising a sensing unit, an actuation unit and a control unit, the actuation unit comprising a robotic arm configured for attachment thereto of an instrument inserted through a guide tube, the sensing unit configured to sense a force acting on the instrument and/or a force acting on the guide tube, the control unit communicatively connected to both the sensing unit and the actuation unit and configured to control positional and postural adjustment of the robotic arm according to the intraoperative remote center of motion adjustment method according to any one of claims 17 to 27 so that the RCM remains unchanged in terms of position and posture with respect to the support apparatus.

30. The remote center of motion adjustment system according to claim 29, further comprising a detection unit which is communicatively connected to the control unit and configured to detect positional and postural variation information of the actuation unit, wherein the control unit is configured to provide closed-loop control over the actuation unit and the detection unit.

31. A surgical robot system, comprising a support apparatus, a guide tube and the remote center of motion adjustment system according to claim 29 or 30, wherein the instrument attached to the robotic arm in the remote center of motion adjustment system is configured to be inserted through the guide tube, and wherein the control unit in the remote center of motion adjustment system is configured to adjust a position and posture of the robotic arm so that the RCM remains unchanged in terms of position and posture with respect to the support apparatus.

EP 4 349 295 A1

Fig. 1

| | |
|---|---|
| ESTABLISH SURGICAL SCENE | SO1 |
| MAKE INCISIONS | SO2 |
| DETERMINE RCMs | SO3 |
| DEFINE SAFETY ZONE | SO4 |
| ADJUST RCMs | SO5 |
| SELF-ADAPTIVE ADJUSTMENT OF ROBOT | SO6 |

Fig. 2

Fig. 3

| | |
|---|---|
| SP1 — DETERMINE WORLD COORDINATES | |
| SP2 — DETERMINE COORDINATES OF PATIENT-SIDE CONTROL DEVICE | SP5 → COORDINATE UNIFICATION |
| SP3 — DETERMINE COORDINATES OF OPERATING TABLE | |
| SP4 — DETERMINE COORDINATES OF RCMs | |

Fig. 4a

| | |
|---|---|
| SP1 — DETERMINE WORLD COORDINATES | |
| SP6 — DETERMINE COORDINATES OF PATIENT-SIDE SURGICAL PLATFORM | SP5 → COORDINATE UNIFICATION |
| SP4 — DETERMINE COORDINATES OF RCMs | |

Fig. 4b

Fig. 5

Fig. 6

Fig. 7a

Fig. 7b

Fig. 8

Fig. 9

Fig. 10

Fig. 11

Fig. 12a

Fig. 12b

Fig. 13

Fig. 14

Fig. 15a

Fig. 15b

Fig. 16

Fig. 17

Fig. 18a

Fig. 18b

Fig. 19a

Fig. 19b

Fig. 20

Fig. 21

810

210

Fig. 22

822

820

821

821

400

Fig. 23

Fig. 24

PROVIDE INDICATION OF WHETHER
POSITION OF PATIENT IS TO BE
ADJUSTED
SC0

DETERMINE EXTERNAL FORCE
SC1

MAKE ADJUSTMENT
SC2

Fig. 25

221

900

410

Fig. 26

Fig. 27a

Fig. 27b

Fig. 28

Fig. 29

Fig. 30

Fig. 31

Fig. 32

Fig. 33

Fig. 34a

Fig. 34b

Fig. 35a

Fig. 35b

Fig. 36

Adjust the patient's position?

| Yes | | No |

Fig. 37

Instrument is being retracted …
Robot is restoring its original
configuration …

Fig. 38a

Instrument has been successfully
retracted. Adjustment is ongoing …

Fig. 38b

Fig. 39

Fig. 40

Fig. 41

Adjustment is completed, and the positions of the instruments have been restored. The surgery can be resumed …

Fig. 42

## INTERNATIONAL SEARCH REPORT

| | |
|---|---|
| | International application No. |
| | **PCT/CN2022/096744** |

**A.    CLASSIFICATION OF SUBJECT MATTER**

A61B 34/30(2016.01)i;  A61B 34/00(2016.01)i

According to International Patent Classification (IPC) or to both national classification and IPC

**B.    FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, ENTXTC, ENTXT, VEN, CJFD: 上海微创医疗机器人, 宋子威, 郑阿勇, 江磊, 何超, 王家寅, 张晓波, 严加强, 外科, 手术, 不动点, RC点, 参考点, 位姿, 阈值, 角度, 距离, 位置, 预定, 力, 传感, 力矩, surgical, operational, fixed point, constraint, limited, reference, RCM, Remote Center of Manipulation, RC point, posture, gesture, location, distance, angle, threshold, limit, predetermined, force, sensor, moment

**C.    DOCUMENTS CONSIDERED TO BE RELEVANT**

| Category* | Citation of document, with indication, where appropriate, of the relevant passages | Relevant to claim No. |
|---|---|---|
| X | CN 107072725 A (INTUITIVE SURGICAL OPERATIONS, INC.) 18 August 2017 (2017-08-18)<br>    description paragraphs 33-155 | 28-31 |
| X | CN 108210070 A (MICROPORT (SHANGHAI) MEDICAL ROBOT CO., LTD.) 29 June 2018 (2018-06-29)<br>    description, paragraphs 52-87 | 28-31 |
| X | US 2017079727 A1 (GLOBUS MEDICAL INC) 23 March 2017 (2017-03-23)<br>    description, paragraphs 25-85 | 28-31 |
| A | CN 112263332 A (MICROPORT (SHANGHAI) MEDICAL ROBOT CO., LTD.) 26 January 2021 (2021-01-26)<br>    description, paragraphs 57-131 | 1-16, 28-31 |
| A | CN 109890295 A (SIEMENS HEALTHCARE GMBH) 14 June 2019 (2019-06-14)<br>    entire document | 1-16, 28-31 |
| A | CN 108289719 A (INTUITIVE SURGICAL OPERATIONS, INC.) 17 July 2018 (2018-07-17)<br>    entire document | 1-16, 28-31 |

☑ Further documents are listed in the continuation of Box C.    ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **19 August 2022** | **26 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/096744**

**C.    DOCUMENTS CONSIDERED TO BE RELEVANT**

| Category* | Citation of document, with indication, where appropriate, of the relevant passages | Relevant to claim No. |
|---|---|---|
| A | US 2017245951 A1 (GLOBUS MEDICAL INC) 31 August 2017 (2017-08-31) entire document | 1-16, 28-31 |
| A | WO 2021058294 A1 (KONINKLIJKE PHILIPS N.V.) 01 April 2021 (2021-04-01) entire document | 1-16, 28-31 |

Form PCT/ISA/210 (second sheet) (January 2015)

**INTERNATIONAL SEARCH REPORT**

International application No.

**PCT/CN2022/096744**

| **Box No. II** | **Observations where certain claims were found unsearchable (Continuation of item 2 of first sheet)** |

This international search report has not been established in respect of certain claims under Article 17(2)(a) for the following reasons:

1. ☑ Claims Nos.: **17-27**
because they relate to subject matter not required to be searched by this Authority, namely:

[1] Claims 17-27 relate to an adjustment method for an intraoperative fixed point. Hence, the method is actually an interventional treatment for a human or animal body during surgery, and is essentially a surgical method. Therefore, said claims belong to the subject matter as defined in PCT Rule 39.1(vi) that does not warrant a search conducted by the international searching authority.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

<table>
<tr><td colspan="2" align="center">INTERNATIONAL SEARCH REPORT<br>Information on patent family members</td><td colspan="2">International application No.<br><br>**PCT/CN2022/096744**</td></tr>
</table>

| Patent document cited in search report | | | Publication date (day/month/year) | Patent family member(s) | | | Publication date (day/month/year) |
|---|---|---|---|---|---|---|---|
| CN | 107072725 | A | 18 August 2017 | WO | 2016069648 | A1 | 06 May 2016 |
| | | | | US | 2019142533 | A1 | 16 May 2019 |
| | | | | JP | 2017537663 | A | 21 December 2017 |
| | | | | CN | 110478036 | A | 22 November 2019 |
| | | | | EP | 3212149 | A1 | 06 September 2017 |
| | | | | KR | 20170077109 | A | 05 July 2017 |
| | | | | JP | 2022068243 | A | 09 May 2022 |
| | | | | US | 2021212780 | A1 | 15 July 2021 |
| | | | | JP | 2020096886 | A | 25 June 2020 |
| | | | | US | 2017333141 | A1 | 23 November 2017 |
| CN | 108210070 | A | 29 June 2018 | | None | | |
| US | 2017079727 | A1 | 23 March 2017 | JP | 2018094404 | A | 21 June 2018 |
| | | | | HK | 1254282 | A1 | 19 July 2019 |
| | | | | EP | 3332706 | A1 | 13 June 2018 |
| CN | 112263332 | A | 26 January 2021 | | None | | |
| CN | 109890295 | A | 14 June 2019 | DE | 102016221222 | A1 | 03 May 2018 |
| | | | | US | 2019261932 | A1 | 29 August 2019 |
| | | | | WO | 2018077604 | A1 | 03 May 2018 |
| CN | 108289719 | A | 17 July 2018 | EP | 3405134 | A1 | 28 November 2018 |
| | | | | CN | 113633326 | A | 12 November 2021 |
| | | | | JP | 2019508075 | A | 28 March 2019 |
| | | | | KR | 20180097633 | A | 31 August 2018 |
| | | | | US | 2022125532 | A1 | 28 April 2022 |
| | | | | US | 2019008599 | A1 | 10 January 2019 |
| | | | | WO | 2017127202 | A1 | 27 July 2017 |
| US | 2017245951 | A1 | 31 August 2017 | US | 2017239006 | A1 | 24 August 2017 |
| | | | | US | 2017239003 | A1 | 24 August 2017 |
| | | | | US | 2017239007 | A1 | 24 August 2017 |
| | | | | US | 2020155243 | A1 | 21 May 2020 |
| | | | | US | 2018000546 | A1 | 04 January 2018 |
| | | | | US | 2021022814 | A1 | 28 January 2021 |
| | | | | US | 2017304013 | A1 | 26 October 2017 |
| | | | | JP | 2015528713 | A | 01 October 2015 |
| | | | | US | 2020179065 | A1 | 11 June 2020 |
| | | | | US | 2017360517 | A1 | 21 December 2017 |
| | | | | US | 2017281145 | A1 | 05 October 2017 |
| | | | | US | 2017265949 | A1 | 21 September 2017 |
| | | | | US | 2017231702 | A1 | 17 August 2017 |
| | | | | US | 2017252112 | A1 | 07 September 2017 |
| | | | | US | 2017245944 | A1 | 31 August 2017 |
| | | | | US | 2017258533 | A1 | 14 September 2017 |
| | | | | EP | 2863827 | A1 | 29 April 2015 |
| | | | | US | 2014378999 | A1 | 25 December 2014 |
| | | | | US | 2017239002 | A1 | 24 August 2017 |
| | | | | US | 2013345718 | A1 | 26 December 2013 |
| | | | | US | 2022233262 | A1 | 28 July 2022 |
| | | | | WO | 2013192598 | A1 | 27 December 2013 |
| | | | | US | 2019167362 | A1 | 06 June 2019 |
| WO | 2021058294 | A1 | 01 April 2021 | | None | | |

Form PCT/ISA/210 (patent family annex) (January 2015)